(19) Europäisches Patentamt
European Patent Office
Office européen des brevets

(11) EP 3 513 193 B1

(12) EUROPEAN PATENT SPECIFICATION

(45) Date of publication and mention
of the grant of the patent:
07.05.2025 Bulletin 2025/19

(51) International Patent Classification (IPC):
G16H 50/30 (2018.01)       G16H 50/70 (2018.01)

(21) Application number: 17768442.0

(22) Date of filing: 15.09.2017

(52) Cooperative Patent Classification (CPC):
G01N 33/576; G01N 33/92; G16H 10/40;
G16H 50/30; G16H 50/70; Y02A 90/10

(86) International application number:
PCT/EP2017/073241

(87) International publication number:
WO 2018/050804 (22.03.2018 Gazette 2018/12)

(54) **METHOD OF DIAGNOSIS OF NON-ALCOHOLIC FATTY LIVER DISEASES**

VERFAHREN ZUR DIAGNOSE NICHTALKOHOLISCHER FETTLEBERERKRANKUNGEN

PROCÉDÉ DE DIAGNOSTIC DE STÉATOSES HÉPATIQUES D'ORIGINE NON ALCOOLIQUE

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR

(30) Priority: 16.09.2016 EP 16306186

(43) Date of publication of application:
24.07.2019 Bulletin 2019/30

(73) Proprietors:
• Biopredictive
75007 Paris (FR)
• Assistance Publique Hôpitaux de Paris
75012 Paris (FR)

(72) Inventor: POYNARD, Thierry
75006 Paris (FR)

(74) Representative: Flesselles, Bruno F.G.
BF IP
36 rue Jean de la Fontaine
75016 Paris (FR)

(56) References cited:
WO-A1-2006/082522    WO-A2-2006/103570

• POYNARD THIERRY ET AL: "The diagnostic value of biomarkers (SteatoTest) for the prediction of liver steatosis", COMPARATIVE HEPATOLOGY, BIOMED CENTRAL LTD, GB, vol. 4, no. 1, 23 December 2005 (2005-12-23), pages 10, XP021008456, ISSN: 1476-5926, DOI: 10.1186/1476-5926-4-10
• POYNARD THIERRY ET AL: "Diagnostic value of biochemical markers (NashTest) for the prediction of non alcoholo steato hepatitis in patients with non-alcoholic fatty liver disease", BMC GASTROENTEROLOGY, BIOMED CENTRAL LTD., LONDON, GB, vol. 6, no. 1, 10 November 2006 (2006-11-10), pages 34, XP021022807, ISSN: 1471-230X, DOI: 10.1186/1471-230X-6-34
• THIERRY POYNARD ET AL: "Performance of Biomarkers FibroTest, ActiTest, SteatoTest, and NashTest in Patients with Severe Obesity: Meta Analysis of Individual Patient Data", PLOS ONE, vol. 7, no. 3, 14 March 2012 (2012-03-14), pages e30325, XP055327259, DOI: 10.1371/journal.pone.0030325
• M. MUNTEANU ET AL: "Diagnostic performance of FibroTest, SteatoTest and ActiTest in patients with NAFLD using the SAF score as histological reference", ALIMENTARY PHARMACOLOGY & THERAPEUTICS., vol. 44, no. 8, 23 August 2016 (2016-08-23), GB, pages 877 - 889, XP055327194, ISSN: 0269-2813, DOI: 10.1111/apt.13770

## Description

The invention relates to a new non-invasive quantitative test making it possible to detect and help characterize subjects at risk of non-alcoholic fatty liver disease (NAFLD) and in particular those with non-alcoholic steato-hepatitis (NASH).

The tests as disclosed in the present application make it possible, in particular to identify among subjects at risk of non-alcoholic fatty liver disease (NAFLD),

  ◦ those with non-alcoholic steato-hepatitis (NASH) and
  ◦ those with clinically significant NAFLD disease (significant inflammation including NASH or significant fibrosis).

The solution provided by the present application lies in two new tests

  ◦ a non-invasive quantitative test (NIT) which makes it possible to determine whether a person at risk is to be treated or followed-up, assessing the severity of inflammation, independently of steatosis and fibrosis,
  ◦ a binary test which predicts histological significant NAFLD disease, combining inflammation (activity) and fibrosis and uses markers used in the NIT as disclosed above.

## Background of the invention

Non-alcoholic fatty liver disease (NAFLD) is one of the causes of fatty liver, occurring when fat is deposited (steatosis) in the liver due to causes other than excessive alcohol use. NAFLD is the most common liver disorder in developed countries. NAFLD is a condition generally associated with factors of the metabolic syndrome.

While a liver may remain fatty without disturbing liver function, it may also progress to become non-alcoholic steatohepatitis (NASH), a state in which steatosis is combined with inflammation and fibrosis (steatohepatitis). NASH is a progressive disease: over a 10-year period, up to 20% of patients with NASH will develop cirrhosis of the liver, and 10% will suffer death related to liver disease.

Non-alcoholic steatohepatitis (NASH) is thus the most extreme form of NAFLD, and is regarded as a major cause of cirrhosis of the liver of unknown cause.

As indicated in Ratziu et al (2007, Aliment Pharmacol Ther 25, 207-218), and Perazzo et al (2014, Aliment Pharmacol Ther 40:1081-93), there is a strong association between the number of metabolic risk factors and the prevalence of significant fibrosis presumed by FibroTest, which predicted overall survival in patients with type-2 diabetes and/or dyslipidemia.

In order to improve the outcome of the disease, it is important to be able to detect it in the patients as early as possible as the outcome is good if the condition is detected and treated in its early stages. However, it is impossible to perform liver biopsy in such a large number of patients with metabolic risk factors to identify patients with advanced liver fibrosis or with steatohepatitis (non-alcoholic steatohepatitis, NASH).

It is thus important to be able to detect, especially among these patients, those having a liver disease (activity grade A2 or above or fibrosis stage F2 or above (according to SAF classification)) in order to be able to treat these patients or follow them up. Such detection should be reliable, non-invasive and as little expensive as possible as it is to be made on a large number of patients.

Current methods of diagnosis are measurement of elevated liver enzymes and/or liver ultrasound showing steatosis. An ultrasound may also be used to exclude gallstone problems (cholelithiasis). Liver biopsy (tissue examination) is the test widely accepted as definitively distinguishing NASH from other forms of liver disease and can be used to assess the severity of the inflammation (activity scores) and resultant fibrosis.

Non-invasive diagnostic tests have been developed, such as FibroTest, that estimates liver fibrosis (Halfon et al, 2008), and SteatoTest, that estimates steatosis (Ratziu et al, 2006), however their use has not been widely adopted (Vuppalanchi and Chalasani, 2009). Apoptosis has been indicated as a potential mechanism of hepatocyte injury as caspase-cleaved cytokeratin 18 (M30-Apoptosense ELISA) in serum/plasma is often elevated in patients with NASH and tests based on these parameters have been developed (Sowa et al, 2013). However, as the role of oncotic necrosis has yet to be examined it is unknown to what degree apoptosis acts as the predominant form of injury (Feldstein et al, 2009; Musso et al, 2010).

Other diagnostic tests are available. Relevant blood tests include erythrocyte sedimentation rate, glucose, albumin, and kidney function. Because the liver is important for making proteins used in coagulation some coagulation related studies are often carried out especially the INR (international normalized ratio). In people with fatty liver with associated inflammatory injury (steatohepatitis) blood tests are usually used to rule out viral hepatitis (hepatitis A, B, C and herpes viruses like EBV or CMV), rubella, and autoimmune related diseases. Hypothyroidism is more prevalent in NASH patients which would be detected by determining the TSH (Liangpunsakul and Chalasani, 2003).

It has been suggested that in cases involving overweight patients whose blood tests do not improve on losing weight and exercising that a further search of other underlying causes is undertaken. This would also apply to those with fatty liver who are very young or not overweight or insulin-resistant. In addition, those whose physical appearance indicates the possibility of a congenital syndrome, have a family history of liver disease, have abnormalities in other organs, and those that present with moderate to advanced fibrosis or cirrhosis (Cassiman and Jaeken, 2008).

Previous most used non-invasive quantitative test (NIT) in patients with presumed NAFLD had limitations, including validations using histological reference mixing fibrosis, steatosis or activity (NAFLDscore, FIB4) or not continuous quantitative score (NashTest, BARD), and are mostly developed for Fibrosis (NAFLDscore, FIB4).

NashTest, developed by the BioPredictive (Paris, France) is diagnostic for non-alcoholic steato hepatitis (NASH) in patients with metabolic steatosis (overweight, diabetes, hyperlipidemia) and combines alpha2-macroglobulin, haptoglobin, apolipoprotein A1, total bilirubin, GGT, fasting glucose, triglycerides, cholesterol, ALT and AST, with parameters adjusted for patient's age, gender, weight and height.

The NAFLDscore (Angulo et al, Hepatology. 2007 Apr;45(4):846-54) uses the markers age, hyperglycemia, body mass index, platelet count, albumin, and AST/ALT ratio.

The BARD score (Cichoz-Lach et al, Med Sci Monit. 2012; 18(12): CR735-CR740) was composed of 3 variables: AST/ALT ratio $\geq$0.8 (2 points); a BMI $\geq$28 (1 point); and the presence of diabetes (1 point). The possible score ranges from 0 to 4 points. According to the results of Harrison et al., BARD scores equaling 0 or 1 are of high (96%) negative predictive value (NPV) for advanced fibrosis (Gut. 2008;57:1441-47).

The FIB4 index is based on age, aspartate and alanine aminotransferase and platelet counts. The formula is ( Age x AST ) / ( Platelets x ( sqr ( ALT )).

However, so far, none of the above NITs makes it possible to detect patients with liver inflammation or significant NAFLD disease among the "at risk" population, and in particular NASH as histologically defined by Bedossa et al, using FLIP algorithm and SAF scoring system. (Hepatology 2012, Nov;56(5):1751-9; Hepatology 2014, Aug;60(2):565-75).

Furthermore, the previous NITs as disclosed above were not constructed according to several variability factors, that took into account the variability of histological references and the choices of appropriate cutoffs for biomarkers.

Poynard et al. (2005, Comp. Hepatol. 4, 10) disclose SteatoTest as a non-invasive quantitative estimate of liver steatosis. Steatotest uses Body Mass Index and fasting glucose as biological markers.

Poynard et al. (2006, BMC Gastroenterol. 6, 34) disclose NashTest as a simple and non-invasive biomarker reliably predicts the presence or absence of NASH, that uses glucose, height and weight of the patient.

WO2006082522 relates to a method for detecting the extent of hepatic steatosis in a patient, by using the serum concentration of biological markers. Such method isn't able to detect specifically NASH and proposed to use BMI.

WO2006103570 relates to a method for detecting the extent of alcoholic steato-hepatitis (ASH) or non-alcoholic steato-hepatitis (NASH) in a patient, by using the serum concentration of biological markers. The definition of NASH is different from the definition of NASH of the present application and thus the method will not identify the same patients.

Poynard et al. (2012, PLOS One 7, e30325) perform an overview of 3 studies which assessed the performance of non-invasive markers of fibrosis (FibroTest), steatosis (SteatoTest) and steato-hepatitis (NashTest, ActiTest) for assessing liver lesions in patients with severe obesity.

In view of the above, it is thus necessary to develop a new test that would make it possible to reliably detect liver disease and the need to engage in treatment or follow-up of a patient, in particular in people presenting a factor of metabolic syndrome (who are the people most at risk of having developing NAFLD), while limiting the risk of false-positive or false-negative hits.

The problem with people with metabolic syndrome is that all don't present a liver dysfunction that would lead to development of NAFLD and ultimately to NASH. As indicated above, in view of the number of people "at risk", and of the risks associated with liver biopsy, it is further not possible to perform liver biopsies on all patients with a factor of metabolic syndrome (or presenting liver steatosis as detected by echography) in order to see whether they would present a NAFLD symptom and need treatment or follow-up.

It is further to be noted that there is no current gold standard to detect whether people have a NAFLD, as diagnostic is ultimately made by the physician in view of the observations made by the histologist, which are subject of variability linked to the samples actually observed and the classification eventually decided by the observer, also the SAF classification has greatly improved the classification and stratification of patients.

## Metabolic Syndrome

Metabolic syndrome is associated with the risk of developing cardiovascular disease and type 2 diabetes. Some studies have shown the prevalence in the USA to be an estimated 34% of the adult population, and the prevalence increases with age.

Metabolic syndrome is the name for a group of risk factors that increases the risk for the patient to develop heart disease (such as coronary heart disease where plaque builds up inside the coronary arteries, chest pain, heart attack, heart damage, myocardial infarction...) and other health problems such as diabetes and strokes.

These factors are

(1) Abdominal (central) obesity (apple shape): overweight with adipose tissue accumulation particularly around the waist and trunk. Excess fat in the stomach area is a greater risk factor for heart disease than excess fat in other parts of the body, such as on the hips

(2) High serum triglycerides (without treatment)

(3) Low high-density lipoprotein (HDL) cholesterol level (without treatment) HDL cholesterol helps clear whole cholesterol from arteries. A low HDL cholesterol level raises your risk for heart disease.

(4) Elevated blood pressure (without treatment). Blood pressure is the force of blood pushing against the walls of arteries as the heart pumps blood. If this pressure rises and stays high over time, it can damage the heart and lead to plaque buildup.

(5) Elevated fasting plasma glucose (without treatment), insulin resistance, or prediabetes. This may be an early sign of diabetes.

In the present context, one would consider that a patient is "at risk" and thus within the population where the tests herein described are of great interest, if he presents at least one, or more generally at least two, preferably at least three of these five factors.

Clinical manifestations of metabolic syndrome could also include:

(6) Chest pains or shortness of breath: Suggesting the rise of cardiovascular and other complications

(7) *Acanthosis nigricans,* hirsutism, peripheral neuropathy, and retinopathy, in particular in patients with insulin resistance and hyperglycemia or with diabetes mellitus

(8) *Xanthomas* or *xanthelasmas,* especially in patients with severe dyslipidemia

(9) Hyperuricemia

(10) polycystic ovarian syndrome (in women)

(11) erectile dysfunction (in men)

Consequently, a patient considered "at risk" in the present context, *i.e.* a patient for which there is a benefit to perform the tests as herein described is a patient presenting at least one, preferably at least two, more preferably at least three of the clinical features (1) to (11), and more preferably at least one of the clinical features (1) to (5), more preferably at least two, more preferably at least three of the clinical features (1) to (5).

The applicant followed an original and innovative strategy that made it possible to identify some biochemical markers and to design a quantitative test and a qualitative test. This strategy was, in particular, based on a new definition of the patients for which treatment is to be initiated, or who need close follow-up, that differs from the one currently used in the art.

One must understand that the definition of NASH has evolved over time. It was first proposed that NASH patient would present macrovesicular steatosis, hepatocellular ballooning, lobular inflammation that included a component of PMN leukocytes and fibrosis.

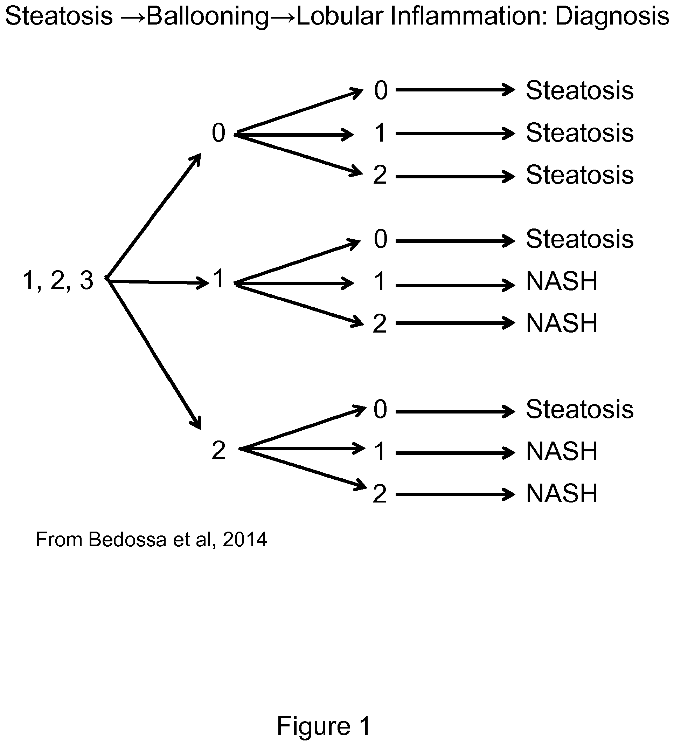

It was then proposed that the main markers of NASH are macrovesicular steatosis (at least 5 % of steatosis, as determined by the SAF method developed by Bedossa et al (see above)), hepatocellular ballooning, lobular inflammation that included a component of PMN leukocytes (Brunt et al, Int. J. Mol. Sci. 2016, 17, 97). Assessing the histological data of patients, the FLIP algorithm (Figure 1) provides a method to determine whether a patient presents a NASH or not (*i.e.* whether treatment or follow-up should be initiated). However, the FLIP algorithm requires that the patient presents a Steatosis SAF-grade S1 or more, thereby excluding patients with SAF-grade S0 who could however present an elevated Activity SAF-grade, lobular inflammation or hepatocyte ballooning.

The inventor used original histological reference for the diagnosis of NASH and was able to demonstrate that it is important to rather focus on ballooning and lobular inflammation, that are specific features of necro-inflammatory histological activity.

In fact, by taking in to account, or not, the various factors that are considered as being important for NASH, the inventor noted that presence of steatosis is not a factor that is important to detect patients in need of treatment or follow-up but.

The inventor was thus able to design a new activity test that particularly adapted for patients "at risk", and in particular more adapted than the non-invasive test already disclosed in the prior art, ActiTest (Biopredictive, Paris, France). The ActiTest is calculated using an original combination of six biochemical markers (alpha2-macroglobulin, haptoglobin, apolipoprotein A1, total bilirubin, GGT, and ALT, parameters adjusted for patient's age and gender), and makes it possible to obtain an index of necro inflammatory activity. However, since this test was designed in and validated for patients with chronic hepatitis C or B, who present a high level of inflammatory activity, it is not sensible enough to be used as routine diagnostic in patients as herein contemplated, as the level of activity in these patients is usually not high enough to be detected by ActiTest.

The inventor thus proposes a new paradigm to decide if a patient presents a significant liver disease and if he must be treated or particularly followed-up:

This new paradigm is equivalent to a new definition of that that would be that a patient is presenting a NASH if

(1) the context is favorable: the patient presents at least one factor of the metabolic syndrome, and the patient doesn't present another liver risk factor (such as viral hepatitis or alcoholism)
(2) the patient presents an Activity equal or higher than grade 2 according to the SAF scoring system:

a. lobular inflammation (presence of necro-inflammatory lesions of grade $\geq$ 2 or
b. hepatocyte ballooning of grade $\geq$ 2, or
c. ballooning=1 and lobular inflammation=1

This definition of a NASH disease is thus different from the one provided by Bedossa et al with the FLIP algorithm, as it doesn't take into account whether the patient has liver steatosis. It is also easier than the NASH definition according to the FLIP algorithm, which has multiple branches of decisions (see Figure 1) and may not be easily workable. Finally, by not using liver steatosis as a criteria for NASH (and a criteria to initiate follow-up or treatment), this definition would not let aside patient with significant activity (grade $\geq$ 2) or significant fibrosis (stage $\geq$ 2) but no steatosis.

Currently, the patients with significant activity or fibrosis but no steatosis, or patients with high lobular inflammation but no ballooning (or the opposite), may not be treated, as not included in the NASH definition that is currently recognized in the art. The new definition of NASH is thus more sensitive than the previous definition of NASH according to the FLIP algorithm that is considered to lead to a high number of false-negative patients that are, however, of clinical significance.

The "Metabolic-NASH-ActiTest" as designed by the inventor and herein described makes it possible to detect patients that present these characteristics and that can thus be qualified as NASH patients. It is to be noted that the test is a quantitative test as will be described below.

Using the same innovative definition of NASH (in particular exclusion of the factor "steatosis" from the definition of the disease) and the same markers that were used for designing the "Metabolic-NASH-ActiTest" disclosed below, the inventor was able to design a further test that permits to predict significant NAFLD disease as defined by the SAF scoring system (A2 or F2) ("Metabolic-FibroActiTest").

In the context of the present disclosure, except if specifically indicated, inflammation, steatosis grades or fibrosis stages are evaluated using the SAF classification as disclosed in the art and reminded in Example 1. The term "significant" is indicated for a stage or grade equal or higher than 2. Inflammation and activity are used interchangeably in the context of the present disclosure.

In developing the assays herein described, the inventor selected a new combination of 11 components, among 13 components possibly associated with NAFLD. In particular, the inventor was able to exclude the fasting glucose which need fasting before harvest of blood or plasma samples, as well as BMI (Body Mass Index) the reliability of which is arguable with several causes of assessment variability, both for height and weight measurements. The fact that these two factors (that are used in the BARD score, NashTest and NAFLDscore) are excluded from the test herein disclosed will clearly simplify the access to care, reproducibility of the data and thus reliability of the conclusions that can been drawn, only relying of directly and unambiguously measurable markers.

Finally, and most importantly, for the construction of the new test, various factors of variability were taken into account:

(a) the choice of histological cutoff (percentage of hepatocytes with steatosis) for defining steatosis,
(b) the inclusion or not of steatosis in the definition of NASH,
(c) the inclusion of histological controls without NAFLD, without steatosis and activity.

Using this methodology, which had not been described before, to the knowledge of the applicant, made it possible to obtain a very robust test that would lead to reliable results and information.

In summary, the design of the tests as herein disclosed was made using various non-common features:

- a new definition of NASH that redefines the patients that need to be treated or followed-up
- exclusion of BMI and fasting glucose from the studied markers, in order to improve ease of use and reliability of the tests
- verification of the robustness of the test, by multiple tests by varying parameters
- inclusion of data from patients that had liver disease, but no steatosis or activity which permitted to increase the size of a control group for assessing the robustness of the tests specificity, that were generally not included in the design of the tests already disclosed

The present application thus discloses two tests useful for taking decision for clinicians. It is indeed to be noted that these tests provide information (an index) that the physician will use, in combination with the clinical context, to decide whether to further investigate the clinical state of the patient, whether to initiate treatment, or whether the patient's state doesn't necessitate treatment.

The applicant thus designed a first test that may be called "Metabolic-NASH-ActiTest", a quantitative NIT (the output of which ranges from 0.00 to 1) of the necroinflammatory histological activity.

This test makes it possible to predict the presence of histological activity specific to NASH (ballooning and/or lobular inflammation) and combines the following 11 components:

(1) alpha2-macroglobulin (A2M) (g / l)
(2) Age (years)
(3) alanine aminotransferase (ALT) (IU / l)
(4) apolipoprotein A-1 (Apoa1) (g / l)
(5) Aspartate transaminase (also named aspartate aminotransferase, AST) (IU / l)
(6) total bilirubin (BILI) ($\mu$mol/l)
(7) cholesterol (CT) (mmol/l)
(8) Gender (0 for women, 1 for men)
(9) gamma-glutamyl transpeptidase (GGT) (IU / l)
(10) Haptoglobin (Hapto) (g / l)
(11) Triglycerides (TG) (mmol/l).

The second test may be called "Metabolic-FibroActiTest" and is a binary test, permitting to predict significant liver disease (activity grade 2 or fibrosis stage 2) or mild NAFLD disease (no activity grade 2 and no fibrosis grade 2) and uses the same markers.

This second test can be written using the FibroTest®, a NIT extensively validated for the diagnostic and prognosis of fibrosis stages, as one of its components, with 4 other components associated not included in the FibroTest® panel but included in the "Metabolic-NASH-ActiTest" as disclosed herein: ALT, AST, cholesterol and triglycerides. This choice allowed to simplify the drafting of the "Metabolic-FibroActiTest" while preventing a co-linearity effect as some components of the Metabolic-NASH-ActiTest were already included in the FibroTest components: apolipoprotein A1, haptoglobin, alpha-2-macroglobulin, total bilirubin, GGT, age and gender.

This second test "Metabolic-FibroActiTest" share the common feature with the first test "Metabolic-NASH-ActiTest" as having been designed using the same innovative definition of liver disease that doesn't make steatosis a prerequisite for a patient to be included in the definition.

It is reminded that FibroTest® is a universal validated fibrosis test widely disclosed in the art, and in particular in WO 2002/016949, and is marketed by the applicant Biopredictive (Paris, France).

The quality of a diagnosis test would generally be determined by drawing a Receiving Operating Characteristic (ROC) curve and measuring the Area Under Receiving Operating Characteristic curve (AUROC).

The ROC curve is drawn by plotting the sensitivity versus (1-specificity), after classification of the patients, according to the result obtained for the diagnosis test, for different thresholds (from 0 to 1).

It is usually acknowledged that a ROC curve the area under which has a value superior to 0.7 is a good predictive curve for diagnosis. The ROC curve has to be acknowledged as a curve allowing prediction of the quality of a diagnosis test. It is best for the AUROC to be as closed as 1 as possible, this value describing a test which is 100 % specific and sensitive.

It is reminded that

(1) sensitivity is the probability that the diagnosis is positive in individuals suffering from the disease sought (detection of true positives): the test is positive if the patient is suffering from the disease. The sensitivity is low when the number of false negatives is high. The sensitivity is calculated by the formula SE = (number of individuals suffering from the disease in whom the sign is present)/(number of individuals suffering from the disease in whom the sign is present + number of individuals suffering from the disease in whom the sign is absent).

(2) specificity is the probability that the diagnosis is negative in the individuals not suffering from the disease sought (non-detection of true negatives): the test is negative if the patient is not suffering from the disease. The specificity is low when the number of false positives is high. The specificity is calculated by the formula SP = (number of individuals not suffering from the disease in whom the sign is absent)/(number of individuals not suffering from the disease in whom the sign is absent + number of individuals not suffering from the disease in whom the sign is present).

(3) Positive predictive value (PPV): is the probability of having the disease if the diagnostic test is positive (i.e. that the patient is not a false positive): the patient is suffering from the disease if the test is positive. The positive predictive value is calculated by the formula PPV = (number of individuals suffering from the disease in whom the sign is present)/(number of individuals suffering from the disease in whom the sign is present + number of individuals not suffering from the disease in whom the sign is present).

(4) Negative predictive value (NPV): is the probability of not having the disease if the diagnostic test is negative (that the patient is not a false negative): the patient is not suffering from the disease if the test is negative. The negative predictive value is calculated by the formula NPV = (number of individuals not suffering from the disease in whom the sign is absent)/(number of individuals not suffering from the disease in whom the sign is absent + number of individuals suffering from the disease in whom the sign is absent)

In order to obtain a good diagnostic test, it is important to both increase specificity and sensitivity.

In developing the assays and tests as herein disclosed, the inventor increased the sensitivity of the test by defining NASH by the presence of either ballooning of the hepatocyte or lobular inflammation (rather than by the presence of these two elements further in the presence of steatosis). The inventor showed that a better AUROC was obtained by not using steatosis as a criterion for NASH.

Specificity was increased and verified by using "strong" negative controls such as patients who don't *a priori* have a liver condition (such as patients A0F0 according to the METAVIR classification) or may have presented a liver condition (such as liver activity or liver fibrosis) but that was clearly not linked to fatty disease (such as patients cured from hepatitis C, who don't have a metabolic syndrome: although these patients may have presented a beginning of fibrosis or liver activity that is not linked to NAFLD or NASH, they would thus be qualified as negative in the assay as herein disclosed).

Generally, a diagnosis (or diagnostic) method comprises

i. a step of gathering information from the patient
ii. a step of comparing said information with regards to thresholds
iii. a step of deducing, from the difference between the patient's information and the threshold, whether the patient has a specific disease or the stage of the patient's disease.

As a matter of illustration

i. the information that can be gathered from the patient can be gathered directly from the patient (such as images from NMR, scanner, radiography, contrast-enhanced computed tomography), or indirectly from the patient, such as from a biological sample that has been obtained from a patient (such as urine, blood sample..). The information can be presence (or absence) and/or level of specific biological markers, whether specific from the pathogenic determinant (bacterial or viral DNA), or elevated levels of patient's markers
ii. once the information is obtained, it is compared to different values / standards and the deviation with regards to these standards is assessed. As a matter of illustration, the level of some biomarkers shall be compared to the level usually observed in healthy patients and to the levels usually observed in patients with the disease. Thresholds may exist, where 95 % of patients having passed the threshold have the disease and 95 % of the patients not having passed the threshold do not have the disease. For diseases where multiple clinical stages can be determined, such thresholds can discriminate the different stages. In this step ii, one may compare various types of information to their respective standards, in order to be able to reach a diagnostic in step iii (as a matter of illustration, one can use the values and information obtained from measurement of various blood or plasma markers, images from scanner and of Body Mass Index).
iii. the last step is actually making the diagnosis i.e. deciding whether or not the patient has the condition sought, taking, in particular, into account the information gathered from the patient, the thresholds as described above. The physician may also take into account other elements (such as the consistency of the information gathered or the like) to make the diagnostic.

Some diagnostic methods, such as the ones disclosed in the present application, shall also include a step i.a), which comprise the steps of modifying the information obtained from the patient in order to obtain a new type of information, which is the one that is then compared to the standards in step ii.

It is further to be noted that the mere measurement of the values of levels of markers in the plasma or serum of a patient and the combination thereof in an algorithm as herein disclosed is part of a diagnostic method but only provides an intermediate result (an index) that would then to be compared to a reference index, in order to actually be able to pose the diagnostic.

It is also to be noted that the tests herein disclosed are not "gold-standard" tests, in the sense that the output (index calculated by the formulas herein disclosed) isn't a definitive answer as to the state of the patient. Indeed, these tests are based on statistics and there may thus be false-positive or false-negative results, which is the reason why the specific experience of the physician in interpreting the index is of importance for reaching a diagnosis.

However, due to the specificity, sensitivity, positive predictive value and negative predictive value of the tests, herein provided for various thresholds of the index, these tests are of great interest in provided a help to the physician when investigating a clinical case. Consequently, step iii as disclosed above is not direct and immediate from step ii, as the physician must interpret the result from the clinical and general context to be able to reach a diagnosis.

The invention relates to a method for developing a non-invasive diagnosis test (comprising a logistic function, obtained by logistic regression analysis) for detecting NASH in a patient, or for obtaining a logistic function that can be used in a non-invasive diagnosis test for detecting NASH in a patient, wherein said logistic function combines the values of the concentration of biochemical markers in the serum of said patient, comprising the steps of:

   a) classifying patients of a cohort of patients into different groups according to the presence of NASH as determined by analysis of liver biopsy, wherein a patient is classified as having NASH if he

      i. has at least one factor of the metabolic syndrome, and not any other chronic or acute liver disease and
      ii. presents hepatocyte ballooning or lobular activity (at least grade 1 each or at least grade 2 for one)

   b) identifying, among the biochemical markers, the value of which has been measured, the ones which differ significantly between these groups by unidimensional analysis
   c) performing a logistic regression analysis to assess the independent discriminative value of these markers identified in step b) for the diagnosis of NASH
   d) constructing the logistic function by combination of these identified independent factors,

wherein the biochemical markers the concentration of which is measured are chosen in the group consisting of $\alpha$2-macroglobulin, AST (aspartate aminotransferase), ALT (alanine aminotransferase), GGT (gammaglutamyl transpeptidase), total bilirubin, haptoglobin, apoA1, triglycerides, total cholesterol, $\gamma$-globulin, albumin, $\alpha$1-globulin, $\alpha$2-globulin, $\beta$-globulin, IL10, TGF-$\beta$1, apoA2, apoB, cytokeratin 18, platelets number, prothrombin level, hyaluronic acid, urea, N-terminal of type III pro-collagen, Tissue inhibitor metalloproteinase type-1 (TIMP-1), type IV collagen (Coll IV) and osteoprotegerin, wherein the logistic function does not comprise BMI and fasting glucose, wherein the logistic function further includes gender and age of the patient.

The same steps can be applied in a method for developing a non-invasive test that can be used for reaching the diagnosis of NASH in a patient, which is also encompassed within the invention. this test will help the physician reach the diagnosis

This method is thus based on the new definition of NASH by the inventor, as indicated above.

Presence of an acute or chronic liver disease in a patient may lead to an erroneous diagnosis when applying the NITs disclosed herein. Indeed, even if the patient presents a factor of metabolic syndrome, concomitant presence of the liver disease would likely shift the result of the test. Conclusion on the presence of NASH would thus be drawn for patient without such disease.

An acute or chronic liver disease that must be taken into consideration is preferably included in the group consisting of chronic hepatitis B, chronic hepatitis C, alcoholic liver disease (including liver fibrosis or cirrhosis), auto-immune hepatitis, primary biliary cirrhosis, primary sclerosing cholangitis, extra hepatic cholestasis, liver cancer with necrosis or cholestasis, infectious cholangitis, acute steatosis of pregnancy, hepatitis related to medication, iron overload (hemochromatosis), Wilson disease and alpha 1-antitrypsin deficiency. Other liver diseases may be taken into consideration by the physician in order to decide the weight to give to the results of the tests herein disclosed and the conclusion to reach with regards to the NASH status of the patient.

Classification in step a) is performed by histological analysis of liver samples that have been obtained by liver biopsy for each patient of the cohort. The different groups are "patient with NASH disease" and "patient without NASH disease". As indicated above, patients that are classified in the first group are the ones that present either hepatocyte ballooning or lobular activity, with an activity grade (sum of the grade of ballooning and of the grade of lobular inflammation according to the SAF classification) equal or higher than 2.

Presence of liver steatosis alone is not considered as a criterion sufficient for inclusion within the "NASH disease" group. Absence of liver steatosis or level of steatosis below 1 % or 5 % is not considered as a criterion sufficient to exclude a patient from the "NASH disease" group if the patient presents either one of hepatocyte ballooning grade 2 or lobular activity grade 2, or at least grade 1 for both ballooning and lobular inflammation and has at least one factor of the metabolic syndrome.

In the method for developing such diagnostic test, one would prefer one or more of the following, in either combination:

(a) in step a), to use a test cohort comprising at least 100 patients.
(b) In the cohort of a), that at least 50% of the patients should be presenting at least one factor of metabolic syndrome (and thus at risk of having NASH), and not an exclusion factor (chronic or acute liver disease)
(c) In the cohort of a), that at least 10% of the patients are patients who have no steatosis and no activity
(d) The biochemical markers the concentration of which is measured are chosen in the group consisting of α2-macroglobulin, AST (aspartate aminotransferase), ALT (alanine aminotransferase), GGT (gammaglutamyl transpeptidase), total bilirubin, haptoglobin, apoA1, triglycerides, total cholesterol, fasting glucose, γ-globulin, albumin, α1-globulin, α2-globulin, β-globulin, IL10, TGF-β1, apoA2, apoB, cytokeratin 18 and cytokeratin 19 components, platelets number, prothrombin level, hyaluronic acid, urea, N-terminal of type III pro-collagen, Tissue inhibitor metalloproteinase type-1 (TIMP-1), type IV collagen (Coll IV) and osteoprotegerin
(e) The biochemical markers the concentration of which is measured are chosen in the group consisting of α2-macroglobulin, AST (aspartate aminotransferase), ALT (alanine aminotransferase), GGT (gammaglutamyl transpeptidase), total bilirubin, haptoglobin, apoA1, triglycerides, total cholesterol
(f) at least 6, more preferably at least 7, more preferably at least 8, more preferably at least 9, biochemical markers are used in the logistic function of d)
(g) The method further comprises a step of validating the logistic function on a validation cohort comprising at least 100 patients;

The inventor showed below that it is possible not to include BMI and fasting glucose.

**Metabolic-NASH-ActiTest**

The invention relates to an *in vitro* method for diagnosis of NASH in a patient comprising the step of:

a) combining the values as measured from markers present in the serum or plasma of said patient through a logistic function, wherein said first logistic function is a1 + a2 x Log (A2M, g/l) + a3 x Age (years) + a4 x Log (ALT, IU / l) + a5 x (Apoa1, g/l) + a6 x Log (AST, IU/l) + a7 x Log (BILI, μmol/l) + a8 x Log (CT, mmol/l) + a9 x Gender (0 for women, 1 for men) + a10 x Log(GGT, IU / l) + a11 x Log (Hapto, g/l) + a12 x Log (TG, mmol/l)

With

$$-8 \leq a1 \leq -7$$

- 0.1 ≤ a2 ≤ 0.6 preferably 0.15 ≤ a2 ≤ 0.55
- 0.02 ≤ a3 ≤ 0.05 preferably 0.03 ≤ a3 ≤ 0.04
- 1.1 ≤ a4 ≤ 1.5 preferably 1.2 ≤ a4 ≤ 1.4

$$-0.2 \leq a5 \leq 1.0$$

- 1.8 ≤ a6 ≤ 2.3 preferably 1.95 ≤ a6 ≤ 2.2
- 0.8 ≤ a7 ≤ 1.6 preferably 0.9 ≤ a7 ≤ 1.5
- -1.7 ≤ a8 ≤ -1.3 preferably -1.6 ≤ a8 ≤ -1.4

$$0.015 \leq a9 \leq 0.20$$

- 0.15 ≤ a10 ≤ 0.25 preferably 0.20 ≤ a10 ≤ 0.22
- -0.3 ≤ a11 ≤ 0.1
- 0.9 ≤ a12 ≤ 1.2 preferably 1.0 ≤ a12 ≤ 1.1

In particular said logistic function is:

-7.82349 + 0.50879 x Log (A2M, g/l) + 0.036625 x Age (years) + 1.22544 x Log (ALT, IU/l) -0.12954 x (Apoa1, g/l) + 2.18581 x Log (AST, IU/l) + 1.48183 x Log (BILI, μmol/l) -1.49351 x Log (CT, mmol/l) + 0.019536 Gender (0 for women, 1 for men) + 0.21614 x Log(GGT, IU / l) -0.026321 x Log (Hapto, g/l) + 1.09487 x Log (TG, mmol/l).

In another embodiment, said function is -7.370196 + 0.18026 x Log (A2M, g/l) + 0.034609 x Age (years) + 1.47222 x Log (ALT, IU / l) + 0.089966 x (Apoa1, g/l) + 1.99317 x Log (AST, IU/l) + 0.98523 x Log (BILI, $\mu$mol/l) -1.55580 x Log (CT, mmol/l) + 0.17857 x Gender (0 for women, 1 for men) + 0.020437 x Log(GGT, IU / l) + 0.055873 x Log (Hapto, g/l) + 1.00712 x Log (TG, mmol/l).

The coefficients of the formulas as provided above have been rounded to the 5$^{th}$ decimal.

Said method may also include the steps of obtaining (measuring) the different values used in the logistic function, such as the steps of measuring, in a blood or a plasma sample that have previously been harvested from the patient, the concentration / values / quantities of the various biological markers as mentioned above.

Said method may also include the steps of comparing the first index to a predetermined threshold, in particular to determine whether it is higher or lower than said threshold.

Said method may also include the step of deducing the presence of NASH in said patient if the first index is above said threshold.

The test as designed here is a quantitative test, the first index value ranging from 0 to 1. It is considered that a patient is likely to present a NASH disease (according to the definition herein provided) and would thus need a treatment or follow-up when the first index is equal or above 0.5.

It is to be noted that the test is quantitative. Consequently, the higher is the First Index, the more severe is the NASH disease. When the value of the First Index is around 0.5, the table above help take a decision on the diagnosis, but the general and specific clinical contexts must also be taken into consideration.

The invention also encompasses an *in vitro* method for obtaining a first index comprising the step of:

i. combining the values as measured from markers present in the serum or plasma of a patient through a logistic function, wherein said first logistic function is a1 + a2 x Log (A2M, g/l) + a3 x Age (years) + a4 x Log (ALT, IU / l) + a5 x (Apoa1, g/l) + a6 x Log (AST, IU/l + a7 x Log (BILI, $\mu$mol/l) + a8 x Log (CT, mmol/l) + a9 x Gender (0 for women, 1 for men) + a10 x Log(GGT, IU / l) + a11 x Log (Hapto, g/l) + a12 x Log (TG, mmol/l)

With

$$-8 \leq a1 \leq -7$$

- $0.1 \leq a2 \leq 0.6$ preferably $0.15 \leq a2 \leq 0.55$
- $0.02 \leq a3 \leq 0.05$ preferably $0.03 \leq a3 \leq 0.04$
- $1.1 \leq a4 \leq 1.5$ preferably $1.2 \leq a4 \leq 1.4$

$$-0.2 \leq a5 \leq 1.0$$

- $1.8 \leq a6 \leq 2.3$ preferably $1.95 \leq a6 \leq 2.2$
- $0.8 \leq a7 \leq 1.6$ preferably $0.9 \leq a7 \leq 1.5$
- $-1.7 \leq a8 \leq -1.3$ preferably $-1.6 \leq a8 \leq -1.4$

$$0.015 \leq a9 \leq 0.20$$

- $0.15 \leq a10 \leq 0.25$ preferably $0.20 \leq a10 \leq 0.22$

$$-0.3 \leq a11 \leq 0.1$$

- $0.9 \leq a12 \leq 1.2$ preferably $1.0 \leq a12 \leq 1.1$

Preferred logistic functions for this embodiment are disclosed above.

The first index is usable for in a diagnostic method and will be used by the physician to eventually decide whether the patient presents or not a NASH, whether the patient needs to be treated or not, or further investigated. The decision from the physician will depend on the value of the index, as well as on other factors (general and specific medical context).

The present specification also discloses a device for diagnosis of NASH in a patient, comprising:

a) a first means, wherein the first means provides a first index by combining the values as measured from markers present in the serum or plasma of a patient through a logistic function, wherein said first logistic function is a1 + a2 x Log (A2M, g/l) + a3 x Age (years) + a4 x Log (ALT, IU / l) + a5 x (Apoa1, g/l) + a6 x Log (AST, IU/l) + a7 x Log (BILI, $\mu$mol/l) + a8 x Log (CT, mmol/l) + a9 x Gender (0 for women, 1 for men) + a10 x Log(GGT, IU / l) + a11 x Log (Hapto, g/l) + a12 x Log (TG, mmol/l)

With

$$-8 \leq a1 \leq -7$$

- $0.1 \leq a2 \leq 0.6$ preferably $0.15 \leq a2 \leq 0.55$
- $0.02 \leq a3 \leq 0.05$ preferably $0.03 \leq a3 \leq 0.04$
- $1.1 \leq a4 \leq 1.5$ preferably $1.2 \leq a4 \leq 1.4$

$$-0.2 \leq a5 \leq 1.0$$

- $1.8 \leq a6 \leq 2.3$ preferably $1.95 \leq a6 \leq 2.2$
- $0.8 \leq a7 \leq 1.6$ preferably $0.9 \leq a7 \leq 1.5$
- $-1.7 \leq a8 \leq -1.3$ preferably $-1.6 \leq a8 \leq -1.4$

$$0.015 \leq a9 \leq 0.20$$

- $0.15 \leq a10 \leq 0.25$ preferably $0.20 \leq a10 \leq 0.22$

$$-0.3 \leq a11 \leq 0.1$$

- $0.9 \leq a12 \leq 1.2$ preferably $1.0 \leq a12 \leq 1.1$.

Said first index can be later used to determine the presence of NASH in said patient and whether it is necessary to initiate treatment or follow-up, in particular using the help of table 1 above.

In a specific embodiment, the first mean is computerized. It may be an electronic spreadsheet with the formula recorded within, that provides the first index as an output when entering the various elements mentioned above. It can also be a computer program that provides the first index as an output after receipt of the various elements mentioned above.

The first means can present one or more of the following, in either combination:

- Operate within a private or public network
- Receive the inputs (values of the various elements mentioned above) from a sender that is in a remote place (i.e. they are sent to the first means from a different location that where the first means is located)
- Require the sender to identify himself before sending the inputs
- Receive the inputs (values of the various elements mentioned above) from a secure manner
- Send the output (first index) to the sender of the inputs
- Store the output in a database (possibly with a unique identifier, making it possible to assign inputs, outputs to this identifier)
- Provides the first index with further information (such as sensitivity and/or specificity and/or positive predictive value and/or negative predictive value linked to the prevalence of the condition in the population to which belongs the patient)

Is also foreseen a non-transitory computer readable storage medium, having stored thereon a computer program comprising program instructions, the computer program being loadable into a data-processing unit and adapted to cause the data-processing unit to carry out a method for calculating a first index by combining the values as measured from markers present in the serum or plasma of a patient through a logistic function as disclosed above, when the computer program is run by the data-processing device.

**Metabolic-FibroActiTest**

In the context of the present disclosure, a "significant" or "severe" NAFLD is defined as NAFLD with a SAF-Activity score $\geq 2$ and/or a SAF-Fibrosis score $\geq 2$.

The invention also relates to an *in vitro* method for diagnosis of severity of NAFLD disease in a patient comprising the steps of :

a) combining the values as measured from markers present in the serum or plasma of said patient through a logistic function, wherein said first logistic function is

b1 + b2 x Log (A2M, g/l) + b3 x Age (years) + b4 x Log (ALT, IU / l) + b5 x (Apoa1, g/l) + b6 x Log (AST, IU/l) + b7 x Log (BILI, $\mu$mol/l) + b8 x Log (CT, mmol/l) + b9 x Gender (0 for women, 1 for men) + b10 x Log(GGT, IU / l) + b11 x Log (Hapto, g/l) + b12 x Log (TG, mmol/l) in order to obtain a first index
With

- -28 $\leq$ b1 $\leq$ -22 preferably -26 $\leq$ b1 $\leq$ -25
- 13 $\leq$ b2 $\leq$ 19 preferably 15 $\leq$ b2 $\leq$ 17
- 0.05 $\leq$ b3 $\leq$ 0.15 preferably 0.08 $\leq$ a3 $\leq$ 0.12
- 0.9 $\leq$ b4 $\leq$ 1.4 preferably 1.0 $\leq$ b4 $\leq$ 1.2

$$-4.4 \leq b5 \leq -4.1$$

- 2.4 $\leq$ b6 $\leq$ 2.7 preferably 2.5 $\leq$ b6 $\leq$ 2.6
- 6.0 $\leq$ b7 $\leq$ 6.4 preferably 6.2 $\leq$ b7 $\leq$ 6.3
- -0.8 $\leq$ b8 $\leq$ -0.4 preferably -0.7 $\leq$ b8 $\leq$ -0.5

$$1.0 \leq b9 \leq 1.1$$

- 3.4 $\leq$ b10 $\leq$ 3.8 preferably 3.6 $\leq$ b10 $\leq$ 3.7
- -5.0 $\leq$ b11 $\leq$ -4.5 preferably -4.9 $\leq$ b11 $\leq$ -4.8
- 1.0 $\leq$ b12 $\leq$ 1.2 preferably 1.05 $\leq$ b12 $\leq$ 1.15

A preferred Metabolic-FibroActiTest formula is - 25.98652 + 16.00374 x Log(Alpha2Macroglobulin (g/l)) + 0.10067 x Age (in years) + 1.12881 x Log (ALT, IU / l) - 4.24187 x ApoA1 (g/l) + 2.55422 x Log (AST, IU / l) + 6.22308 x Log(Bilirubin ($\mu$mol/l)) + 0.59340 x Log (CT, mmol/l) + 1.07838 x Sex (female=0, male=1) + 3.64357 x Log(GGT (IU/l)) - 4.86167 x Log(Haptoglobin (g/l)) + 1.11641 Log (TG, mmol/l).

The coefficients of the formula as provided above have been rounded to the 5th decimal.

The above Metabolic-FibroActiTest can also be written as -6.13858 + 1.12881 x Log (ALT, IU / l) + 2.55422 x Log (AST, IU / l) - 0.59340 x Log (CT, mmol/l) + 3.58266 x (4.467 x Log(Alpha2Macroglobulin (g/l)) - 1.357 x Log(Haptoglobin (g/l)) + 1.017 x Log(GGT (IU/l)) + 0.0281 x Age (in years) + 1.737 x Log(Bilirubin ($\mu$mol/l)) - 1.184 x ApoA1 (g/l) + 0.301 x Sex (female=0, male=1) -5.540) + 1.11641 Log (TG, mmol/l).

Consequently, the above Metabolic-FibroActiTest can also written as -6.13858 + 1.12881 x Log (ALT, IU / l) + 2.55422 x Log (AST, IU / l) - 0.59340 x Log (CT, mmol/l) + 3.58266 x Fibrotest + 1.11641 Log (TG, mmol/l) where "Fibrotest" represents the value obtained after applying the Fibrotest® test to the patient.

It is reminded that the formula for Fibrotest®, disclosed in WO 2002/016949, is 4.467 x Log(Alpha2Macroglobulin (g/l)) - 1.357 x Log(Haptoglobin (g/l)) + 1.017 x Log(GGT (IU/l)) + 0.0281 x Age (in years) + 1.737 x Log(Bilirubin ($\mu$mol/l)) - 1.184 x ApoA1 (g/l) + 0.301 x Sex (female=0, male=1) -5.540

The method can thus also be described as comprising a step of combining the values as measured from markers present in the serum or plasma of said patient through a logistic function, wherein said first logistic function is c1 + c2 x Log (ALT, IU / I) + c3 x Log (AST, IU/I) + c4 x Log (CT, mmol/l) + c5 x Fibrotest + c6 x Log (TG, mmol/l) in order to obtain a first index

With

- -7 $\leq$ c1 $\leq$ -5 preferably -6.5 $\leq$ c1 $\leq$ -5.5
- 0.9 $\leq$ c2 $\leq$ 1.4 preferably 1.0 $\leq$ c24 $\leq$ 1.2
- 2.4 $\leq$ c3 $\leq$ 2.7 preferably 2.5 $\leq$ c3 $\leq$ 2.6
- -0.8 $\leq$ c4 $\leq$ -0.4 preferably -0.7 $\leq$ c4 $\leq$ -0.5
- 3.0 $\leq$ c5 $\leq$ 4.2 preferably 3.2 $\leq$ c5 $\leq$ 4.0
- 1.0 $\leq$ c6 $\leq$ 1.2 preferably 1.05 $\leq$ c6 $\leq$ 1.15

Said method may also include the steps of obtaining (measuring) the different values used in the logistic function, such as the steps of measuring, in a blood or a plasma sample that have previously been harvested from the patient, the concentration / values / quantities of the various biological markers as mentioned above.

Said method may also include the steps of comparing the final index to a predetermined threshold, in particular to determine whether it is higher or lower than said threshold.

Said method may also include the step of deducing the severity of NAFLD in said patient if the final index is above said threshold. This deduction step shall be made by the physician, using in particular the information provided in Table 1.

Table 1. Predictive Value Section for Severity using the Empirical ROC Curve for a prevalence of 0.59 (PPV: positive predictive valor; NPV: negative predictive valor).

| Cutoff | | | Likelihood | Prev. | = 0.59 |
|---|---|---|---|---|---|
| Value | Sensitivity | Specificity | Ratio | PPV | NPV |
| 0.40 | 0.812 | 0.688 | 2.606 | 0.790 | 0.718 |
| 0.60 | 0.580 | 0.813 | 3.095 | 0.817 | 0.573 |

In view of this table, it appears that an index below 0.40 would indicate that the patient doesn't present a significant NAFLD (high sensitivity of the test), whereas an index above 0.60 would reflect presence of a significant NAFLD (high specificity of the test). In any case, and as this test is not a gold-standard test, the physician would further use the global and specific clinical context to conclude, and especially when the index is between 0.40 and 0.60.

The invention also encompasses an *in vitro* method for obtaining a first index comprising the step of combining the values as measured from markers present in the serum or plasma of a patient through a logistic function, wherein said first logistic function is as disclosed above (Metabolic-FibroActiTest).

This first index would be used by the physician as a help to determine whether the patient suffers from significant NAFLD. This index is thus later used in a diagnostic method to determine the severity of NAFLD in said patient.

The specification also discloses a device for diagnosis of severity of NAFLD in a patient, comprising:

a) a first means, wherein the first means provides a first index by combining the values as measured from markers present in the serum or plasma of a patient through a logistic function, wherein said first logistic function is as disclosed above (Metabolic-FibroActiTest)

In a specific embodiment, the first mean is computerized. It may be an electronic spreadsheet with the formula recorded within, that provides the first index as an output when entering the various elements mentioned above. It can also be a computer program that provides the first index as an output after receipt of the various elements mentioned above.

The first means can present one or more of the following, in either combination:

- Operate within a private or public network
- Receive the inputs (values of the various elements mentioned above) from a sender that is in a remote place (i.e. they are sent to the first means from a different location that where the first means is located)
- Require the sender to identify himself before sending the inputs
- Receive the inputs (values of the various elements mentioned above) from a secure manner
- Send the output (first index) to the sender of the inputs
- Store the output in a database (possibly with a unique identifier, making it possible to assign inputs, outputs to this identifier)
- Provides the first index with further information (such as sensitivity and/or specificity and/or positive predictive value and/or negative predictive value linked to the prevalence of the condition in the population to which belongs the patient)

Is also foreseen a non-transitory computer readable storage medium, having stored thereon a computer program comprising program instructions, the computer program being loadable into a data-processing unit and adapted to cause the data-processing unit to carry out a method for calculating a first index by combining the values as measured from markers present in the serum or plasma of a patient through a logistic function as disclosed above, when the computer program is run by the data-processing device.

As indicated above, the methods and device herein disclosed are intended to be used, preferably for patients presenting at least one factor of the metabolic syndrome.

It is further to be noted that the methods as disclosed reflect a level of injury of the liver (activity possibly coupled with fibrosis) and that diagnosis may be refined by any other methods, such as a liver biopsy in the patient to determine the exact state of the liver.

The invention is also drawn to a kit of diagnosis of NASH and/or NAFLD in a patient, comprising instructions allowing to determine the presence of liver fibrosis and/or liver cirrhosis lesions in said patient, after dosage of the biochemical markers, and measurement of liver stiffness.

The instructions comprise the various logistic functions that has to be used after determination of the dosage of the biochemical markers. It can appear as a printed support as well as a computer usable support, such as a software. The instructions may also comprise the threshold and tables that permit to obtain the predictive values, depending of the expected prevalence of fibrosis in the patient population. It may also comprise reagents needed to measure the values of the various markers.

The following examples are meant to describe an aspect of invention, but shall not be limiting the invention.

## DESCRIPTION OF THE FIGURES

Figure 1: Diagnostic algorithm for NASH (from Bedossa et al, Hepatology, April 2014; Aug;60(2):565-75; doi: 10.1002/hep.27173)

Figure 2: Definitions of NASH according to the original FLIP algorithm and according to three extended definitions: 27 combinations possible.

Figure 3: A. Comparison of AUROCS between Algo-9, ActiTest and FibroTest. B. Comparison of AUROCS between Algo-9 with standard blood tests: BARD, FIB4 and NAFLD-score

## EXAMPLES

### Example 1. Methods: SAF score and FLIP algorithm

The classification was performed according to the following criteria, with the following paragraphs directly extracted from Bedossa et al (Hepatology, April 2014; Aug;60(2):565-75; doi: 10.1002/hep.27173).

For each biopsy a SAF score (Steatosis, Activity, Fibrosis) summarizing the main histological lesions was defined. This assesses both and separately the grade of steatosis (S), the grade of activity (A) and the stage of fibrosis (F), the latter according to NASH CRN (Kleiner et al Hepatology 2005;41:1313-1321)

Steatosis was assessed by the percentage of hepatocytes containing large and medium-sized intracytoplasmic lipid droplets (but not foamy microvesicles), on a scale of 0 to 3 (S0: <5%; S1: 5%-33%, S2: 34%-66%, S3: >67%).

Ballooning of hepatocytes was graded from 0 to 2 (0: normal hepatocytes with cuboidal shape, sharp angles and pink eosinophilic cytoplasm; 1: presence of clusters of hepatocytes with a rounded shape and pale cytoplasm, usually reticulated where although the shape is different, the size is similar to that of normal hepatocytes; 2, as for grade 1, but where there was also at least one enlarged ballooned hepatocyte (at least 2-fold size compared with that of normal cells within a cluster of hepatocytes with grade 1 ballooning).

Lobular inflammation was defined as a focus of two or more inflammatory cells within the lobule organized either as microgranulomas or located within the sinusoids. Foci were counted at 20 X magnification (grade 0: none; 1: < 2 foci per lobule; 2: >2 foci per lobule).

The grade of activity (A from A0 to A4) was calculated by addition of grades of ballooning and lobular inflammation.

Then, the FLIP algorithm that enables dichotomous classification of a biopsy in either the steatosis without-NASH or the steatosis-with-NASH groups, was used. It is based on the semi-quantification of 3 elementary features, steatosis, clarification/ballooning of hepatocytes and lobular inflammation as evaluated according to the SAF score.

For classification using the FLIP algorithm, steatosis was used as the criterion for entry into the algorithm weighted by hepatocellular ballooning and lobular inflammation. For any biopsy with at least grade 1 steatosis, the algorithm included 9 possible means of arriving at a decision. As described in Figure 1, a case presenting with at least grade 1 of each of the three features (steatosis, ballooning, lobular inflammation) was classified as NASH. Other cases were diagnosed as steatosis (without NASH). Other histologic features possibly present in NASH were not considered for categorization.

## Example 2 - Methodology for developing the NASH-non-invasive test

Recent simplified histological references were used for constructing new NITs, with modifications of these references due to the risk of sampling errors.

All histological scores were made according to the SAF classification as disclosed above.

For the binary histological diagnosis of NASH, it was decided to focus on ballooning and lobular inflammation, the specific features of necro-inflammatory histological activity as suggested by the histological FLIP algorithm. (Bedossa et al. Hepatology 2012, and Bedossa et al. Hepatology 2014).

It was indeed considered that the original FLIP algorithm has several limitations, the arbitrary choice of 5% of hepatocytes steatosis defining presence of steatosis, the exclusion of cases without steatosis for the diagnostic of NASH despite the severe activity grades, and the arbitrary choice of requiring both ballooning and lobular inflammation even if one of this feature reached a grade 2.

These choices underestimate the risk of false negative of a biopsy, which is not a perfect gold standard with significant risk due to sampling error (Ratziu 2005 Gastroenterology. 2005 Jun;128(7):1898-906).

Therefore, the performance of the new NITs was tested not only according to the original FLIP algorithm (named Histo-Algo-V1-S5) as reference, but also according to three other histological algorithms.

For the second algorithm (Histo-Algo-V2-S0), the definition of histological steatosis (for inclusion in the NAS-patients group) was steatosis equal or above 1% (rather than 5% in the original FLIP algorithm).

For the third algorithm, the definition of NASH did not require the presence of histological steatosis (Histo-Algo-V3-Sall-B1-L1).

For the the fourth algorithm (Histo-Algo-V4-Sall-B0F2B2F0), the definition of NASH did not require the presence of histological steatosis and also did not require the presence of both ballooning and lobular inflammation if one of this activity features was scored at least grade-2.

It results that the steatosis definition had two levels (above or the below the threshold that can be 0, 1 or 5 %), the grade of ballooning has 3 levels (SAF classification), the grade of lobular inflammation has 3 levels (SAF classification). Four (4) different algorithms were proposed for the diagnostic of NASH as indicated above;

The details of the 72 possible combinations of histological features (72 combinations= 2 levels x 3 levels x 3 levels x 4 algorithms) are described in Table 2.

Table 2: Details of the 72 possible combinations of histological features: steatosis definition (two levels), grade of Ballooning (3 levels), grade of lobular inflammation (3 levels) and 4 different algorithms proposed for the diagnostic of NASH after histological observation.

| Algorithm | Steatosis (S) | Ballooning | Lobular Inflammation | Classification as NASH or No-NASH based on Histology |
|---|---|---|---|---|
| **Histo-Algo-V1-S5 (Standard reference, FLIP Algorithm)** | | | | |
| | S<5% | 0 | 0 | No-NASH |
| | S<5% | 0 | 1 | No-NASH |
| | S<5% | 0 | 2 | No-NASH |
| | S<5% | 1 | 0 | No-NASH |
| | S<5% | 1 | 1 | No-NASH |
| | S<5% | 1 | 2 | No-NASH |
| | S<5% | 2 | 0 | No-NASH |
| | S<5% | 2 | 1 | No-NASH |
| | S<5% | 2 | 2 | No-NASH |
| | S≥5% | 0 | 0 | No-NASH |
| | S≥5% | 0 | 1 | No-NASH |
| | S≥5% | 0 | 2 | No-NASH |
| | S≥5% | 1 | 0 | No-NASH |
| | S≥5% | 1 | 1 | NASH |

(continued)

| Algorithm | Steatosis (S) | Ballooning | Lobular Inflammation | Classification as NASH or No-NASH based on Histology |
|---|---|---|---|---|
| **Histo-Algo-V1-S5 (Standard reference, FLIP Algorithm)** | | | | |
| | S≥5% | 1 | 2 | NASH |
| | S≥5% | 2 | 0 | No-NASH |
| | S≥5% | 2 | 1 | NASH |
| | S≥5% | 2 | 2 | NASH |
| **Histo-Alao-V2-S0** | | | | |
| | S=0% | 0 | 0 | No-NASH |
| | S=0% | 0 | 1 | No-NASH |
| | S=0% | 0 | 2 | No-NASH |
| | S=0% | 1 | 0 | No-NASH |
| | S=0% | 1 | 1 | No-NASH |
| | S=0% | 1 | 2 | No-NASH |
| | S=0% | 2 | 0 | No-NASH |
| | S=0% | 2 | 1 | No-NASH |
| | S=0% | 2 | 2 | No-NASH |
| | S>0% | 0 | 0 | No-NASH |
| | S>0% | 0 | 1 | No-NASH |
| | S>0% | 0 | 2 | No-NASH |
| | S>0% | 1 | 0 | No-NASH |
| | S>0% | 1 | 1 | NASH |
| | S>0% | 1 | 2 | NASH |
| | S>0% | 2 | 0 | No-NASH |
| | S>0% | 2 | 1 | NASH |
| | S>0% | 2 | 2 | NASH |
| Histo-Algo-V3-noS-B1-L1 | | | | |
| | S<5% | 0 | 0 | No-NASH |
| | S<5% | 0 | 1 | No-NASH |
| | S<5% | 0 | 2 | No-NASH |
| | S<5% | 1 | 0 | No-NASH |
| | S<5% | 1 | 1 | NASH |
| | S<5% | 1 | 2 | NASH |
| | S<5% | 2 | 0 | No-NASH |
| | S<5% | 2 | 1 | NASH |
| | S<5% | 2 | 2 | NASH |
| | S≥5% | 0 | 0 | No-NASH |
| | S≥5% | 0 | 1 | No-NASH |
| | S≥5% | 0 | 2 | No-NASH |
| | S≥5% | 1 | 0 | No-NASH |
| | S≥5% | 1 | 1 | NASH |
| | S≥5% | 1 | 2 | NASH |
| | S≥5% | 2 | 0 | No-NASH |
| | S≥5% | 2 | 1 | NASH |
| | S≥5% | 2 | 2 | NASH |
| Histo-Algo-V4-noS-B0L2-B2L0 | | | | |
| | S<5% | 0 | 0 | No-NASH |
| | S<5% | 0 | 1 | No-NASH |
| | S<5% | 0 | 2 | NASH |

(continued)

| Histo-Algo-V4-noS-B0L2-B2L0 | | | |
|---|---|---|---|
| S<5% | 1 | 0 | No-NASH |
| S<5% | 1 | 1 | NASH |
| S<5% | 1 | 2 | NASH |
| S<5% | 2 | 0 | NASH |
| S<5% | 2 | 1 | NASH |
| S<5% | 2 | 2 | NASH |
| S≥5% | 0 | 0 | No-NASH |
| S≥5% | 0 | 1 | No-NASH |
| S≥5% | 0 | 2 | NASH |
| S≥5% | 1 | 0 | No-NASH |
| S≥5% | 1 | 1 | NASH |
| S≥5% | 1 | 2 | NASH |
| S≥5% | 2 | 0 | NASH |
| S≥5% | 2 | 1 | NASH |
| S≥5% | 2 | 2 | NASH |

72 combinations= 2 levels x 3 levels x 3 levels x 4 algorithms. One will note that the conclusion on presence or absence of NASH is identical for the 9 first lines and the 9 last lines (respectively) of either or Histo-Algo-V3-noS-B1-L1 or Histo-Algo-V4-noS-B0L2-B2L0, due to the fact that presence or absence or steatosis is not taken into account to pose the NASH diagnosis in these histological algorithms.

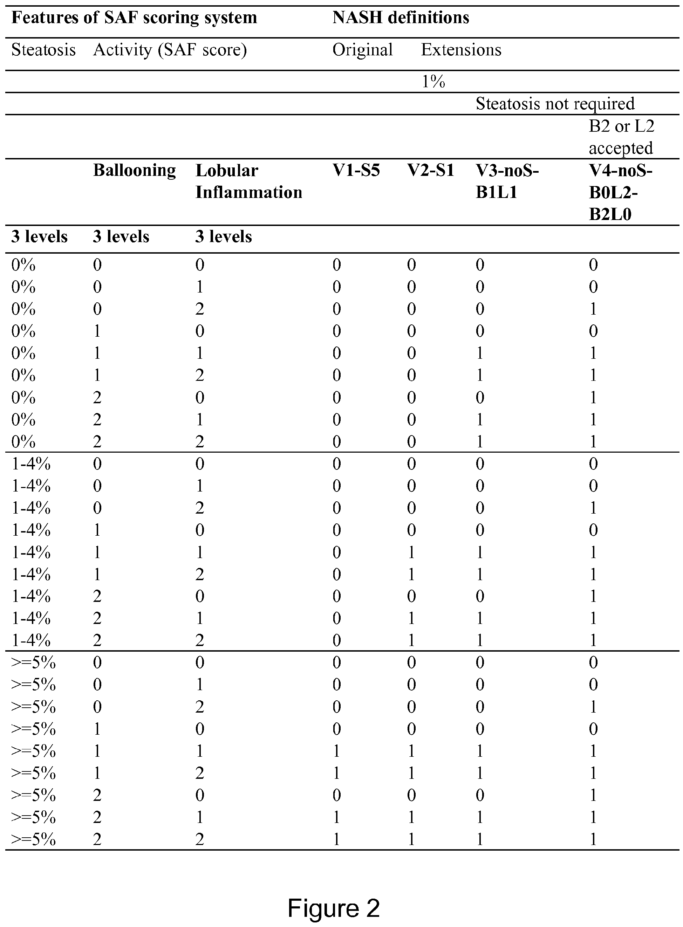

Figure 2 presents a summary of the extension of the definition of NASH, depending on the choice of the histological features taken into account for histological algorithms of Table 2.

## Example 3. Construction of the NIT-Algorithm for the diagnosis of NASH *Choice of the markers to use in these NITs*

It was decided to use ALT, AST, that are known biomarkers of liver necrosis, as well as eight other liver components synthetized by the liver : apolipoprotein A1 (apoA1), haptoglobin, alpha-2-macroglobulin, total bilirubin, GGT, triglycerides, total cholesterol, fasting glucose and three patients characteristics (age, sex, and BMI).

Other markers could be equally used in the procedure herein described, such as markers that have been described in other liver NITs. One could cite γ-globulin, albumin, α1-globulin, α2-globulin, β-globulin, IL10, TGF-β1, apoA2, apoB, cytokeratin 18 and cytokeratin 19 components, platelets number, prothrombin level, hyaluronic acid, urea, N-terminal of type III pro-collagen, Tissue inhibitor metalloproteinase type-1 (TIMP-1), type IV collagen (Coll IV) and osteoprotegerin.

However, it was decided to use only the 13 elements as mentioned above, as the result obtained already provides a test of good quality as observed by the AUROC (see below), and as the markers selected by the inventors seem to be the most relevant in the clinical context. Furthermore, and even though there is no technical difficulties in measuring the plasma level of a large number of biomarkers, it was decided to limit the number of markers used, in particular for economic reasons.

Among the 13 components herein selected serum glucose which requires fasting by the patient for a reliable measure, and BMI which has several causes of measurements variability, are more complicated to collect than the 11 others.

Therefore, for each histological reference, the performances of the NIT-algorithms were significantly reduced using 11 versus 13 components, by modeling NITs that don't include these two markers.

### NIT-Algorithms modeling

Eight NIT-Algorithms were constructed using regression analyses.

These eight NIT-Algorithms are obtained by using either the above 13 or 11 biomarkers and patient's characteristics for each of the 4 histological reference algorithms (that classify the patients as NASH or no-NASH patients, depending on the histological observations, as disclosed in example 2)

- NitAlgo-1-S5-13C was constructed using the original histological FLIP algorithm Histo-Algo-V1-S5 as reference, and using 13 components.

- NitAlgo-2-S5-11C was constructed using the original histological FLIP algorithm Histo-Algo-V1-S5 as reference, and using 11 components.
- NitAlgo-3-S0-13C was constructed using the algorithm Histo-Algo-V2-S0 as reference, and using 13 components.
- NitAlgo-4-S0-11C was constructed using the algorithm Histo-Algo-V2-S0 as reference, and using 11 components.
- NitAlgo-5-noS-13C was constructed using the algorithm Histo-Algo-V3-noS-B1-L1 as reference, and using 13 components.
- NitAlgo-5-noS-11C was constructed using the algorithm Histo-Algo-V3-noS-B1-L1 as reference, and using 11 components.
- NitAlgo-7-noS-13C was constructed using the algorithm Histo-Algo-V4-noS-B0F2-B2F0 as reference, and using 13 components.
- NitAlgo-8-noS-11C was constructed using the algorithm Histo-Algo-V4-noS-B0F2-B2F0 as reference, and using 11 components.

*Results for the NASH-quantitative test for the diagnostic of NASH.*

The performances of the above 8 NIT-algorithms varied not significantly from 0.711 (0.683-0.738) to 0.727 (0.699-0.753) for accuracy as well as for AUROC from 0.779 (0.751-0.807) to 0.796 (0.767-0.821).

Therefore, and for facilitating the access to care in the NAFLD context of use, this validated the choice to design algorithms without fasting glucose and BMI (11 components and not 13).

Algorithm NitAlgo-8-noS-11C presented the highest AUROC (0.796 (95% CI 0.767-0.821)) and Accuracy (0.722 (0.695-0.749)), as compared to the three others algorithms with 11 components (and the highest AUROC out of all the algorithms (13 or 11 components)).

For NitAlgo-8-noS-11C, the histological reference was a definition of NASH which doesn't not require steatosis and takes into account a grade 2 for Activity, either by grade 1 ballooning and lobular inflammation, or by grade 2 either ballooning alone or lobular inflammation alone (this NIT was obtained using the Histo-Algo-V4-noS-B0F2-B2F0 histological classification of patients).

The algorithm NitAlgo-2-S5-11C (which was designed using the histological Histo-Algo-V1-S5 classification of patients) makes it possible to help diagnosing presence or absence or NASH using the standard definition as made by the original and reference FLIP algorithm (requiring at least 5% steatosis and at least grade 1 for ballooning and for lobular inflammation).

Table 3: Regression analyses for the prediction of NASH, including 13 components or 11 components without BMI and fasting glucose.

| Presumed by Blood tests | Biopsy FLIP algorithms | | | Accuracy | AUROC |
|---|---|---|---|---|---|
| | NASH | No-NASH | Total | | |
| **NIT-Algo-1-S5-13C (standard)** | *Histo-Algo-V1-S5* | | | | |
| NASH | 407 | 166 | 573 | 0.715 | 0.785 |
| No-NASH | 142 | 366 | 508 | (0.690-0.745) | (0,757-0.815) |
| Total | 549 | 532 | 1081 | | |
| **NIT-Algo-2-S5-11C** | *Histo-Algo-V1-S5* | | | | |
| NASH | 400 | 158 | 558 | 0.716 | 0.780 |
| No-NASH | 149 | 374 | 523 | (0.688-0.743) | (0.752-0.810) |
| Total | 549 | 532 | 1081 | | |
| **NIT-Algo-3-S0-13C** | *Histo-Algo-V2-S0* | | | | |
| NASH No-NASH | 407 143 | 165 366 | | 0.715 (0.687-0.742) | 0.784 (0.756-0.814) |
| Total | 550 | 531 | 1081 | | |
| **NIT-Algo-4-S0-11C** | *Histo-Algo-V2-S0* | | | | |
| NASH No-NASH | 398 152 | 159 372 | 557 524 | 0.712 (0.684-0.739) | 0.779 (0.751-0.807) |

(continued)

| NIT-Algo-4-S0-11C | *Histo-Algo-V2-S0* | | | | |
|---|---|---|---|---|---|
| Total | 550 | 531 | 1081 | | |
| **NIT-Algo-5-noS-13C** | *Histo-Algo-V3-noS-B1-L1* | | | | |
| NASH | 408 | 163 | 571 | 0.714 | 0.785 |
| No-NASH | 146 | 364 | 510 | (0.686-0.741) | (0.757-0.813) |
| Total | 554 | 527 | 1081 | | |
| **NIT-Algo-6-noS-11C** | *Histo-Algo-V3-noS-B1-L1* | | | | |
| NASH | 400 | 158 | 558 | 0.711 | 0.781 |
| No-NASH | 154 | 369 | 523 | (0.683-0.738) | (0.753-0.809) |
| Total | 554 | 527 | 1081 | | |
| **NIT-Algo-7-noS-13C** | *Histo-Algo-V4-noS-B2L0-B0L2* | | | | |
| NASH | 436 | 143 | 569 | 0.727 | 0.792 |
| No-NASH | 152 | 350 | 512 | (0.699-0.753) | (0.766-0.818) |
| Total | 588 | 493 | 1081 | | |
| **NIT-Algo-8-noS-11C** | *Histo-Algo-V4-noS- B2L0-B0L2* | | | | |
| NASH | 430 | 142 | 572 | 0.722 | 0.792 |
| No-NASH | 158 | 351 | 509 | (0.695-0.749) | (0.767-0.821 0.821) |
| Total | 588 | 493 | 1081 | | |

NIT-Algo-8-noS-11C = -7.82349 + 0.50879 x Log (A2M, g/l) + 0.036625 x Age (years) + 1.22544 x Log (ALT, IU / I) -0.12954 x (Apoa1, g/l) + 2.18581 x Log (AST, IU / I) + 1.48183 x Log (BILI, $\mu$mol/l) -1.49351 x Log (CT, mmol/l) + 0.019536 + 0.21614 x Log(GGT, IU / I) -0.026321 x Log (Hapto, g/l) + 1.09487 x Log (TG, mmol/l).

Specificity, sensitivity, Positive predictive value (PPV) and negative predictive value (NPV) are proposed for three cutoffs (0.25, 0.50 and 0.75) and two prevalence of the disease (0.54 or 0.10), allowing to use the Metabolic-NASH-ActiTest (NIT-Algo-8-noS-11C) as an aid to decision. The corresponding predictive values were given in Tables 4 and 5.

Table 4. ROC Data for Condition = HistoHalgoNASHV4 using the Empirical ROC Curve

| NitAlgo8 Cutoff Value | Count +\|P A | Count +\|A B | Count -\| P C | Count -\| A D | Sensitivity A/(A+C) | False- C/(A+C) | False+ B/(B+D) | Specificity D/(B+D) |
|---|---|---|---|---|---|---|---|---|
| 0.25 | 559 | 289 | 29 | 204 | 0.951 | 0.049 | 0.586 | 0.414 |
| 0.50 | 430 | 142 | 158 | 351 | 0.731 | 0.269 | 0.288 | 0.712 |
| 0.75 | 204 | 45 | 384 | 448 | 0.347 | 0.653 | 0.091 | 0.909 |

Table 5. Predictive Value Section for HistoHalgoNASHV4 using the Empirical ROC Curve

| NitAlgo8 Cutoff Value | Sensitivity | Specificity | Likelihood Ratio | Prev = 0.54 | | Prev. = 0.10 | |
|---|---|---|---|---|---|---|---|
| | | | | PPV | NPV | PPV | NPV |
| 0.25 | 0.951 | 0.414 | 1.622 | 0.659 | 0.876 | 0.153 | 0.987 |
| 0.50 | 0.731 | 0.712 | 2.539 | 0.752 | 0.690 | 0.220 | 0.960 |
| 0.75 | 0.347 | 0.909 | 3.801 | 0.819 | 0.538 | 0.297 | 0.926 |

These tables can be used by the physician to reach a conclusion with regards to the status of the patient.
Another algorithm was developed and is of interest in the present context.

NIT-Algo-2-S5-11C = -7.370196 + 0.18026 x Log (A2M, g/l) + 0.034609 x Age (years) + 1.47222 x Log (ALT, IU /l) + 0.089966 x (Apoa1, g/l) + 1.99317 x Log (AST, IU/l) + 0.98523 x Log (BILI, $\mu$mol/l) -1.55580 x Log (CT, mmol/l) + 0.17857 x --> Gender (0 for women, 1 for men) + 0.020437 x Log(GGT, IU / l) + 0.055873 x Log (Hapto, g/l) + 1.00712 x Log (TG, mmol/l).

This algorithm makes it possible to help diagnosing patients, according to the NASH definition of Bedossa et al (2014).

## Example 5. Methodology for developing the "significant NAFLD" non-invasive test

### Choice of histological reference for the binary diagnosis of "significant NAFLD"

For the binary definition of significant NAFLD, it was decided to rely on the definition as described by Bedossa et al (Bedossa et al Hepatology 2012 and Bedossa et al Hepatology 2014).
This algorithm is simple, a significant NAFLD being the presence of activity SAF-score $\geq$ 2 or a fibrosis SAF-score $\geq$ 2. So far no other NIT has used this histological reference before.

### Definition of significant fibrosis

For the histological diagnosis of significant fibrosis, the reference was the stage 2 being consensual in NAFLD, and similar for the SAF-fibrosis stage (Bedossa Hepatology 2012) than that of NASH-CRN staging (Kleiner Hepatology 2005).

### Definition of significant activity

The FLIP definition of the histological diagnosis of significant activity was different than the FLIP definition of NASH grade-2 (Table 6).
The FLIP definition of the activity SAF-score for "significant activity" was simply the sum of ballooning and lobular-inflammation scores, without requiring presence of steatosis or of the presence of both ballooning and lobular inflammation.
In contrast, the FLIP definition of NASH required the presence of steatosis and presence of both ballooning and lobular inflammation.
Therefore, cases without steatosis (SAF-S0) can be classified as significant NAFLD due to Fibrosis stage F2 (SAF-F2).
Similarly, cases without ballooning but with grade 2 for lobular inflammation (or with ballooning grade 2 and no lobular inflammation) were classified SAF-A2 despite the absence of NASH using the FLIP algorithm.
Therefore, the reference for constructing the best NIT for the prediction of significant NAFLD was the presence of activity SAF-grade $\geq$2 or a fibrosis SAF score $\geq$2 named histological algorithm Histo-Algo-V5-S5-A2-F2.

Table 6: Algorithms for the definition of NASH and significant NAFLD

| Elementary features of NAFLD SAF scoring system | | | | FLIP definition | |
|---|---|---|---|---|---|
| Steatosis | Activity | | Fibrosis | NASH | Significant NAFLD |
| | Ballooning | Lobular Inflammation | | | |
| 2 levels | 3 levels | 3 levels | 2 levels | 2 levels (yes = 1 / no = 0) | 2 levels (yes = 1 / no = 0) |
| <5% | 0 | 0 | <=1 | 0 | 0 |
| <5% | 0 | 0 | >1 | 0 | 1 |
| <5% | 0 | 1 | <=1 | 0 | 0 |
| <5% | 0 | 1 | >1 | 0 | 1 |
| <5% | 0 | >1 | <=1 | 0 | 1 |
| <5% | 0 | >1 | >1 | 0 | 1 |
| <5% | 1 | 0 | <=1 | 0 | 0 |
| <5% | 1 | 0 | >1 | 0 | 1 |

(continued)

| Elementary features of NAFLD SAF scoring system | | | | FLIP definition | |
| Steatosis | Activity | | Fibrosis | NASH | Significant NAFLD |
| | Ballooning | Lobular Inflammation | | | |
| 2 levels | 3 levels | 3 levels | 2 levels | 2 levels (yes = 1 / no = 0) | 2 levels (yes = 1 / no = 0) |
| <5% | 1 | 1 | <=1 | 0 | 1 |
| <5% | 1 | 1 | >1 | 0 | 1 |
| <5% | 1 | >1 | <=1 | 0 | 1 |
| <5% | 1 | >1 | >1 | 0 | 1 |
| <5% | 2 | 0 | <=1 | 0 | 1 |
| <5% | 2 | 0 | >1 | 0 | 1 |
| <5% | 2 | 1 | <=1 | 0 | 1 |
| <5% | 2 | 1 | >1 | 0 | 1 |
| <5% | 2 | >1 | <=1 | 0 | 1 |
| <5% | 2 | >1 | >1 | 0 | 1 |
| >=5% | 0 | 0 | <=1 | 0 | 0 |
| >=5% | 0 | 0 | >1 | 0 | 1 |
| >=5% | 0 | 1 | <=1 | 0 | 0 |
| >=5% | 0 | 1 | >1 | 0 | 1 |
| >=5% | 0 | >1 | <=1 | 0 | 1 |
| >=5% | 0 | >1 | >1 | 0 | 1 |
| >=5% | 1 | 0 | <=1 | 0 | 0 |
| >=5% | 1 | 0 | >1 | 1 | 1 |
| >=5% | 1 | 1 | <=1 | 1 | 1 |
| >=5% | 1 | 1 | >1 | 1 | 1 |
| >=5% | 1 | >1 | <=1 | 1 | 1 |
| >=5% | 1 | >1 | >1 | 1 | 1 |
| >=5% | 2 | 0 | <=1 | 0 | 1 |
| >=5% | 2 | 0 | >1 | 0 | 1 |
| >=5% | 2 | 1 | <=1 | 1 | 1 |
| >=5% | 2 | 1 | >1 | 1 | 1 |
| >=5% | 2 | >1 | <=1 | 1 | 1 |
| >=5% | 2 | >1 | >1 | 1 | 1 |

Among the 36 combinations, 9 (25%) will correspond to the FLIP definition of NASH, and 30 (83%) will correspond to the definition of significant NAFLD.

If Steatosis was defined as 1% and not 5% as defined by the standard algorithm, it is expected that the number of NASH and significant NAFLD cases will increase proportionally to the prevalence of steatosis 1-4% in the population of interest.

## Example 6 Construction of the NIT-Algorithms for the diagnosis of significant NAFLD

### *Choice of components*

The NitAlgo-9 algorithm combined the FibroTest®, a NIT extensively validated for the diagnostic and prognosis of fibrosis stages, with 4 components associated with activity, not included in the FibroTest® panel but included in the NIT-Algorithms for the diagnostic of NASH: ALT, AST, cholesterol and triglycerides (Table 4).

This choice permitted to prevent a co-linearity effect as the other components used in the NASH algorithms described in example 4 were already included in the FibroTest® components: apolipoprotein A1, haptoglobin, alpha-2-macroglobulin, total bilirubin, GGT, age and gender.

*NIT-Algo-9 algorithm modeling.*

The NitAlgo-9 algorithm for the diagnosis of significant NAFLD was constructed using the original FLIP algorithm Histo-Algo-V5-S5-A2-F2 as the reference for histological significant NAFLD, and including 5 components: FibroTest and ALT, AST, cholesterol and triglycerides. (Table 4)

*NAFLD severity algorithm for the prediction of significant NAFLD disease*

According to SAF activity grading the prevalence of histological significant activity (A2A3A4) was 588/1081 = 54.4% and significant fibrosis was 340/1081=31.5%. The prevalence of histological significant NAFLD combining SAF-Activity A2A3A4 or SAF-Fibrosis F2F3F4 = 638/1081 =59.0%.

The performance of the NitAlgo-9 algorithm combining the FibroTest, ALT, AST, cholesterol and triglycerides was highly significant with AUROC= 0.811 and accuracy =0.732 (Table 7).

Table 7: Regression analyses for the prediction of significant NAFLD, including four components: FibroTest, ALT, AST, cholesterol and triglycerides. The histological reference was Histo-Algo-V5- S5A2F2.

| Presumed by Blood tests | Biopsy FLIP algorithms | | | Accuracy | AUROC |
|---|---|---|---|---|---|
| | Significant | Not Significant | Total | | |
| NIT-Algo-9-A2F2 (standard) | Histo-Algo-V5-noS-A2F2 | | | | |
| Significant | 453 | 105 | 558 | 0.732 | 0.814 |
| Not Significant | 185 | 338 | 523 | (0.704-0.758) | (0.786-0.839) |
| Total | 638 | 443 | 1081 | | |

*Performance* of *Comparisons of algorithms with standard comparators: BARD, FIB4 and NAFLD-score* (Table 5)

A total of 574 subjects had simultaneous measurements of NitAlgo-9 algorithm and BARD, FIB4 and NAFLD-score.(Tables 8.A and B)

Table 8.A Empirical Area Under Curve Analysis for Condition = Severity

| Criterion | Empirical Estimate of AUC | AUC's Standard Error | Z-Value to Test AUC > 0.5 | 1-Sided Prob Level | 2-Sided Prob Level | Prevalence Of Severity | Count |
|---|---|---|---|---|---|---|---|
| NAFLDfscore | 0.570 | 0.030 | 2.38 | 0.0086 | 0.0172 | 0.814 | 574 |
| FIB4 | 0.528 | 0.030 | 0.93 | 0.1769 | 0.3538 | 0.814 | 574 |
| BARD | 0.541 | 0.031 | 1.29 | 0.0990 | 0.1980 | 0.814 | 574 |
| Algo9 | 0.675 | 0.029 | 6.14 | 0.0000 | 0.0000 | 0.814 | 574 |

Table 8.B Empirical Test of (AUC1 - AUC2) = 0 for Condition = Severity Table 8: Comparison with other blood tests: BARD, FIB4 and NAFLD-score

| Criterions 1,2 | AUC1 | AUC2 | Value | Difference Std Error | Difference Percent | Z-Value | Prob Level |
|---|---|---|---|---|---|---|---|
| Algo9, NAFLDfscore | 0.675 | 0.570 | 0.105 | 0.040 | -15.54 | 2.63 | 0.0086 |
| Algo9, FIB4 | 0.675 | 0.528 | 0.148 | 0.043 | -21.88 | 3.40 | 0.0007 |
| Algo9, BARD | 0.675 | 0.541 | 0.135 | 0.046 | -19.98 | 2.90 | 0.0037 |

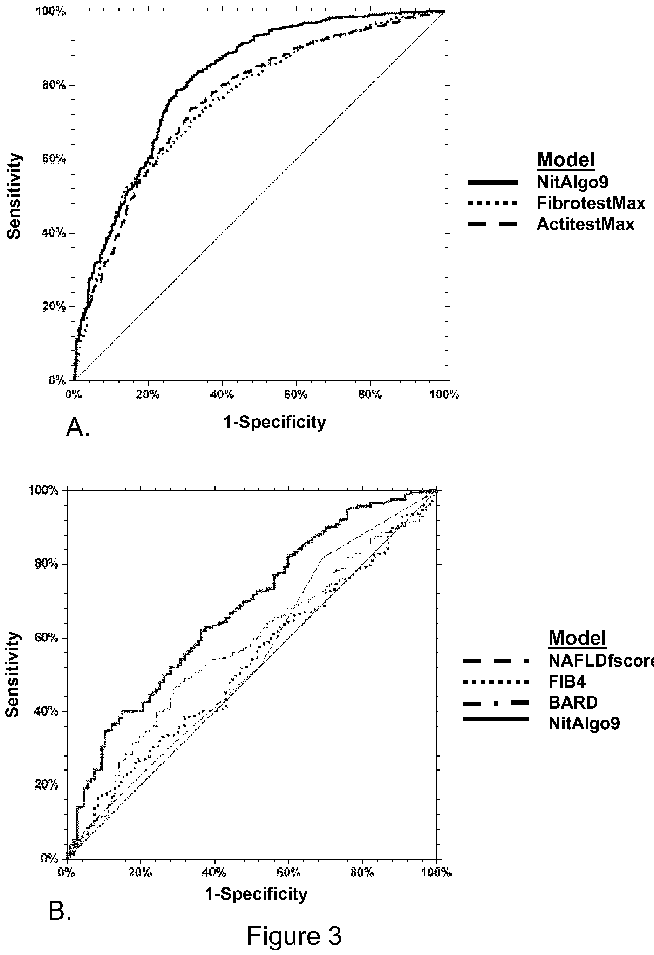

The AUROC of algorithm NitAlgo9 (Metabolic-FibroActiTest) is also better than the one for these tests (Figure 3.B).

The algorithm NitAlgo9 (Metabolic-FibroActiTest) further permitted to obtain significantly higher AUROCs than FibroTest and ActiTest, for the diagnostic of significant Metabolic Liver Disease as defined histologically by Bedossa et (SAF score F2 or A2) (Figure 3.A).

Specificity, sensitivity, Positive predictive value (PPV) and negative predictive value (NPV) are proposed for three cutoffs (0.25, 0.50 and 0.75) and two prevalence of the disease (0.59 or 0.10), to use the Metabolic-FibroActiTest as an aid to decision. The corresponding predictive values were given in Tables 9 and 10 (see also table 1).

Table 9. ROC Data for Condition = Significant disease using the Empirical ROC Curve

| NitAlgo9 Cutoff Value | Count +|P A | Count +|A B | Count -| P C | Count -| A D | Sensitivity A/(A+C) | False- C/(A+C) | False+ B/(B+D) | Specificity D/(B+D) |
|---|---|---|---|---|---|---|---|---|
| 0.25 | 607 | 241 | 31 | 202 | 0.951 | 0.049 | 0.544 | 0.456 |
| 0.50 | 453 | 105 | 185 | 338 | 0.710 | 0.290 | 0.237 | 0.763 |
| 0.75 | 261 | 44 | 377 | 399 | 0.409 | 0.591 | 0.099 | 0.901 |

Table 10. Predictive Value Section for Significant disease using the Empirical ROC Curve

| NitAlgo9 Cutoff Value | Sensitivity | Specificity | Likelihood Ratio | Prev = PPV | 0.59 NPV | Prev. = PPV | 0.10 NPV |
|---|---|---|---|---|---|---|---|
| 0.25 | 0.951 | 0.456 | 1.749 | 0.716 | 0.867 | 0.163 | 0.988 |
| 0.50 | 0.710 | 0.763 | 2.996 | 0.812 | 0.646 | 0.250 | 0.959 |
| 0.75 | 0.409 | 0.901 | 4.119 | 0.856 | 0.514 | 0.314 | 0.932 |

## Example 7 - **Validation of NITs**

The NITs were validated independently in 95 new NAFLD with biopsy and applied in a low risk population of 7,416 healthy volunteers (validation group). They were also applied to 79,955 subjects presumed NAFLD of a USA NASH-FibroSure database.

In the validation group, both NIT-A2F2 and NIT-NASH reached high positive predictive value, 81/85 (95.3%;88.4-98.7) and 82/88 (93.2%;87.9-98.4) for the diagnostic of NASH and significant disease respectively.

According to the definition of the disease, the prevalence of NASH varied (P<0.0001) from 0.8% (n=59) to 17.8% (n=1,317) in the healthy cases, and in USA database from 16.3% (n=13029) to 61.6% (n=49251).

## References

Angulo et al, Hepatology. 2007 Apr;45(4):846-54

Bedossa et al, Hepatology. 2012 Nov;56(5):1751-9. doi: 10.1002/hep.25889.

Bedossa et al, Hepatology, April 2014; Aug;60(2):565-75; doi: 10.1002/hep.27173

Brunt et al, Int. J. Mol. Sci. 2016, 17, 97

Cassiman D, Jaeken J (2008). "NASH may be trash". Gut. 57 (2): 141-4.

Cichoz-Lach et al, Med Sci Monit. 2012; 18(12): CR735-CR740

Feldstein et al (2009). "Cytokeratin-18 fragment levels as noninvasive biomarker for nonalcoholic steatohepatitis: A multicenter validation study". Hepatology. 50 (4): 1072-8.

Halfon et al (2008). "FibroTest-ActiTest as a non-invasive marker of liver fibrosis". Gastroenterol Clin Biol. 32 (6): 22-39.)

Kleiner et al. Design and validation of a histological scoring system for nonalcoholic fatty liver disease. Hepatology 2005;41:1313-1321

Liangpunsakul S, Chalasani N (2003). "Is hypothyroidism a risk factor for non-alcoholic steatohepatitis?". J Clin Gastroenterol. 37 (4): 340-3.

Musso et al (2010). "Meta-analysis: Natural history of non-alcoholic fatty liver disease (NAFLD) and diagnostic accuracy of non-invasive tests for liver disease severity". Annals of Medicine. 43 (8): 1-33

Perazzo et al (2014, Aliment Pharmacol Ther 40:1081-93)

Ratziu et al, 2005 Gastroenterology. 2005 Jun;128(7):1898-906

Ratziu et al (2006). "Diagnostic value of biochemical markers (FibroTest-FibroSURE) for the prediction of liver fibrosis in patients with non-alcoholic fatty liver disease". BMC Gastroenterology. 6: 6.

Ratziu et al, 2007, Aliment Pharmacol Ther 25, 207-218)

Sowa et al (2013). "Novel algorithm for non-invasive assessment of fibrosis in NAFLD.". PLOS ONE. 8 (4)

Vuppalanchi R, Chalasani N (2009). "Non-alcoholic fatty liver disease and non-alcoholic steatohepatitis: Selected practical issues in their evaluation and management". Hepatology. 49 (1): 306-317

**Claims**

1. An *in vitro* method for diagnosis of NASH, Non-alcoholic steato-hepatitis, in a patient comprising the step of:

   a) combining the values as measured from markers present in the serum or plasma of said patient through a logistic function, so as to obtain an index, wherein said index is to be compared to a reference index, wherein said first logistic function is a1 + a2 x Log (alpha2-macroglobulin (A2M), g/l) + a3 x Age (years) + a4 x Log (alanine aminotransferase (ALT), IU / l) + a5 x (apolipoprotein A-1 (Apoa1), g/l) + a6 x Log (Aspartate transaminase (AST), IU/l) + a7 x Log (total bilirubin (BILI), $\mu$mol/l) + a8 x Log (cholesterol (CT), mmol/l) + a9 x Gender (0 for women, 1 for men) + a10 x Log(gamma-glutamyl transpeptidase (GGT), IU / l) + a11 x Log (Haptoglobin (Hapto), g/l) + a12 x Log (Triglycerides (TG), mmol/l) wherein

   i.

   $$-8 \leq a1 \leq -7$$

   ii. $0.1 \leq a2 \leq 0.6$ preferably $0.15 \leq a2 \leq 0.55$
   iii. $0.02 \leq a3 \leq 0.05$ preferably $0.03 \leq a3 \leq 0.04$
   iv. $1.1 \leq a4 \leq 1.5$ preferably $1.2 \leq a4 \leq 1.4$
   v.

   $$-0.2 \leq a5 \leq 1.0$$

   vi. $1.8 \leq a6 \leq 2.3$ preferably $1.95 \leq a6 \leq 2.2$
   vii. $0.8 \leq a7 \leq 1.6$ preferably $0.9 \leq a7 \leq 1.5$
   viii. $-1.7 \leq a8 \leq -1.3$ preferably $-1.6 \leq a8 \leq -1.4$
   ix. $0.015 \leq a9 \leq 0.20$
   x. $0.15 \leq a10 \leq 0.25$ preferably $0.20 \leq a10 \leq 0.22$
   xi.

   $$-0.3 \leq a11 \leq 0.1$$

   xii. $0.9 \leq a12 \leq 1.2$ preferably $1.0 \leq a12 \leq 1.1$.

2. The *in vitro* method of claim 1, wherein the logistic function is -7.82349 + 0.50879 x Log (A2M, g/l) + 0.036625 x Age (years) + 1.22544 x Log (ALT, IU/l) -0.12954 x (Apoa1, g/l) + 2.18581 x Log (AST, IU/l) + 1.48183 x Log (BILI, $\mu$mol/l) -1.49351 x Log (CT, mmol/l) + 0.019536 Gender (0 for women, 1 for men) + 0.21614 x Log(GGT, IU / l) -0.026321 x Log (Hapto, g/l) + 1.09487 x Log (TG, mmol/l).

3. The *in vitro* method of claim 1, wherein the logistic function is -7.370196 + 0.18026 x Log (A2M, g/l) + 0.034609 x Age (years) + 1.47222 x Log (ALT, IU / l) + 0.089966 x (Apoa1, g/l) + 1.99317 x Log (AST, IU/l) + 0.98523 x Log (BILI, $\mu$mol/l) -1.55580 x Log (CT, mmol/l) + 0.17857 x Gender (0 for women, 1 for men) + 0.020437 x Log(GGT, IU / l) + 0.055873 x Log (Hapto, g/l) + 1.00712 x Log (TG, mmol/l).

4. An *in vitro* method for diagnosis of severity of NAFLD, Non-alcoholic fatty liver disease, disease in a patient comprising the steps of :

   a) combining the values as measured from markers present in the serum or plasma of said patient through a logistic function, so as to obtain an index, wherein said index is to be compared to a reference index, wherein said first logistic function is b1 + b2 x Log (alpha2-macroglobulin (A2M), g/l) + b3 x Age (years) + b4 x Log (alanine aminotransferase (ALT), IU / l) + b5 x (apolipoprotein A-1 (Apoa1), g/l) + b6 x Log (Aspartate transaminase (AST), IU/l) + b7 x Log (total bilirubin (BILI), $\mu$mol/l) + b8 x Log (cholesterol (CT), mmol/l) + b9 x Gender (0 for women, 1 for men) + b10 x Log(gamma-glutamyl transpeptidase (GGT), IU / l) + b11 x Log (Haptoglobin (Hapto), g/l) + b12 x Log (Triglycerides (TG), mmol/l) in order to obtain a first index wherein

i. $-28 \leq b1 \leq -22$ preferably $-26 \leq b1 \leq -25$
ii. $13 \leq b2 \leq 19$ preferably $15 \leq b2 \leq 17$
iii. $0.05 \leq b3 \leq 0.15$ preferably $0.08 \leq a3 \leq 0.12$
iv. $0.9 \leq b4 \leq 1.4$ preferably $1.0 \leq b4 \leq 1.2$
v.

$$-4.4 \leq b5 \leq -4.1$$

vi. $2.4 \leq b6 \leq 2.7$ preferably $2.5 \leq b6 \leq 2.6$
vii. $6.0 \leq b7 \leq 6.4$ preferably $6.2 \leq b7 \leq 6.3$
viii. $-0.8 \leq b8 \leq -0.4$ preferably $-0.7 \leq b8 \leq -0.5$
ix.

$$1.0 \leq b9 \leq 1.1$$

x. $3.4 \leq b10 \leq 3.8$ preferably $3.6 \leq b10 \leq 3.7$
xi. $-5.0 \leq b11 \leq -4.5$ preferably $-4.9 \leq b11 \leq -4.8$
xii. $1.0 \leq b12 \leq 1.2$ preferably $1.05 \leq b12 \leq 1.15$.

5. The *in vitro* method of claim 4, wherein the logistic function is $- 25.98652 + 16.00374$ x Log(Alpha2Macroglobulin (g/l)) $+ 0.10067$ x Age (in years) $+ 1.12881$ x Log (ALT, IU / I) $- 4.24187$ x ApoA1 (g/l) $+ 2.55422$ x Log (AST, IU / I) $+ 6.22308$ x Log(Bilirubin ($\mu$mol/l)) $+ 0.59340$ x Log (CT, mmol/l) $+ 1.07838$ x Sex (female=0, male=1) $+ 3.64357$ x Log(GGT (IU/l)) $- 4.86167$ x Log(Haptoglobin (g/l)) $+ 1.11641$ Log (TG, mmol/l).

6. A method for obtaining a logistic function that can be used in a non-invasive

diagnosis test for detecting NASH, Non-alcoholic steato-hepatitis, in a patient, wherein said logistic function combines the values of the concentration of biochemical markers in the serum of said patient, comprising the steps of:

a) classifying patients of a cohort of patients into different groups according to the presence of NASH as determined by analysis of liver biopsy, wherein a patient is classified as having NASH if he

i. has at least one factor of the metabolic syndrome, and not any other chronic or acute liver disease and
ii. presents hepatocyte ballooning or lobular activity (at least grade 1 each or at least grade 2 for one)

b) identifying, among the biochemical markers, the value of which has been measured, the ones which differ significantly between these groups by unidimensional analysis
c) performing a logistic regression analysis to assess the independent discriminative value of these markers identified in step b) for the diagnosis of NASH
d) constructing the logistic function by combination of these identified independent factors,

wherein the biochemical markers the concentration of which is measured are chosen in the group consisting of $\alpha$2-macroglobulin, AST (aspartate aminotransferase), ALT (alanine aminotransferase), GGT (gammaglutamyl transpeptidase), total bilirubin, haptoglobin, apoA1, triglycerides, total cholesterol, $\gamma$-globulin, albumin, $\alpha$1-globulin, $\alpha$2-globulin, $\beta$-globulin, IL10, TGF-$\beta$1, apoA2, apoB, cytokeratin 18, platelets number, prothrombin level, hyaluronic acid, urea, N-terminal of type III pro-collagen, Tissue inhibitor metalloproteinase type-1 (TIMP-1), type IV collagen (Coll IV) and osteoprotegerin, wherein the logistic function does not comprise BMI and fasting glucose,
wherein the logistic function further includes gender and age of the patient.

7. The method of claim 6, wherein the factor of metabolic syndrome is selected from the group consisting of abdominal obesity, high serum triglycerides (without treatment), low high-density lipoprotein (HDL) cholesterol level (without treatment), elevated blood pressure and one of the group consisting of elevated fasting plasma glucose, insulin resistance, and prediabetes.

**EP 3 513 193 B1**

**Patentansprüche**

1. In-vitro-Verfahren zur Diagnose von NASH, nicht-alkoholischer Steato-Hepatitis, bei einem Patienten, umfassend den Schritt:

   a) Kombinieren der Werte, die anhand von im Serum oder Plasma des Patienten vorhandenen Markern gemessen werden, durch eine logistische Funktion, um einen Index zu erhalten, wobei der Index mit einem Referenzindex verglichen werden soll, wobei die erste logistische Funktion a1 + a2 x Log (alpha2-Makroglobulin (A2M), g/l) + a3 x Alter (Jahre) + a4 x Log (Alanin-Aminotransferase (ALT), IU / l) + a5 x (Apolipoprotein A-1 (Apoa1), g/l) + a6 x Log (Aspartat-Transaminase (AST), IU/l) + a7 x Log (Gesamtbilirubin (BILI), $\mu$mol/l) + a8 x Log (Cholesterin (CT), mmol/l) + a9 x Geschlecht (0 für Frauen, 1 für Männer) + a10 x Log(gamma-Glutamyl-Transpeptidase (GGT), IU / l) + a11 x Log (Haptoglobin (Hapto), g/l) + a12 x Log (Triglyceride (TG), mmol/l) wobei

   i.
   $$-8 \leq a1 \leq -7$$

   ii. $0,1 \leq a2 \leq 0,6$ vorzugsweise $0,15 \leq a2 \leq 0,55$
   iii. $0,02 \leq a3 \leq 0,05$ vorzugsweise $0,03 \leq a3 \leq 0,04$
   iv. $1.1 \leq a4 \leq 1.5$ vorzugsweise $1.2 \leq a4 \leq 1.4$
   v.
   $$-0.2 \leq a5 \leq 1.0$$

   vi. $1,8 \leq a6 \leq 2,3$ vorzugsweise $1,95 \leq a6 \leq 2,2$
   vii. $0,8 \leq a7 \leq 1,6$ vorzugsweise $0,9 \leq a7 \leq 1,5$
   viii. $-1,7 \leq a8 \leq -1,3$ vorzugsweise $-1,6 \leq a8 \leq -1,4$
   ix.
   $$0.015 \leq a9 \leq 0.20$$

   x. $0,15 \leq a10 \leq 0,25$ vorzugsweise $0,20 \leq a10 \leq 0,22$
   xi.
   $$-0.3 \leq a11 \leq 0.1$$

   xii. $0,9 \leq a12 \leq 1,2$ vorzugsweise $1,0 \leq a12 \leq 1,1$.

2. *In* vitro-Verfahren nach Anspruch 1, wobei die logistische Funktion -7,82349 + 0,50879 x Log (A2M, g/l) + 0,036625 x Alter (Jahre) + 1,22544 x Log (ALT, IU/l) -0,12954 x (Apoa1, g/l) + 2,18581 x Log (AST, IU/l) + 1.48183 x Log (BILI, $\mu$mol/l) -1.49351 x Log (CT, mmol/l) + 0.019536 Geschlecht (0 für Frauen, 1 für Männer) + 0.21614 x Log(GGT, IU / l) -0.026321 x Log (Hapto, g/l) + 1.09487 x Log (TG, mmol/l).

3. *In* vitro-Verfahren nach Anspruch 1, wobei die logistische Funktion -7,370196 + 0,18026 x Log (A2M, g/l) + 0,034609 x Alter (Jahre) + 1,47222 x Log (ALT, IU / l) + 0,089966 x (Apoa1, g/l) + 1,99317 x Log (AST, IU/l) + 0.98523 x Log (BILI, $\mu$mol/l) -1,55580 x Log (CT, mmol/l) + 0,17857 x Geschlecht (0 für Frauen, 1 für Männer) + 0,020437 x Log(GGT, IU / l) + 0,055873 x Log (Hapto, g/l) + 1,00712 x Log (TG, mmol/l).

4. In-vitro-Verfahren zur Diagnose des Schweregrads einer NAFLD, einer nichtalkoholischen Fettlebererkrankung, bei einem Patienten, das die folgenden Schritte umfasst:

   a) Kombinieren der Werte, die anhand von im Serum oder Plasma des Patienten vorhandenen Markern gemessen werden, durch eine logistische Funktion, um einen Index zu erhalten, wobei der Index mit einem Referenzindex verglichen werden soll, wobei die erste logistische Funktion b1 + b2 x Log (alpha2-Makroglobulin (A2M), g/l) + b3 x Alter (Jahre) + b4 x Log (Alanin-Aminotransferase (ALT), IU / l) + b5 x (Apolipoprotein A-1 (Apoa1), g/l) + b6 x Log (Aspartat-Transaminase (AST), IU/l) + b7 x Log (Gesamtbilirubin (BILI), $\mu$mol/l) + b8 x Log (Cholesterin (CT), mmol/l) + b9 x Geschlecht (0 für Frauen, 1 für Männer) + b10 x Log(Gamma-Glutamyl-Transpeptidase (GGT), IU / l) + b11 x Log (Haptoglobin (Hapto), g/l) + b12 x Log (Triglyceride (TG), mmol/l), um einen ersten Index zu erhalten, wobei

i. $-28 \leq b1 \leq -22$ vorzugsweise $-26 \leq b1 \leq -25$
ii. $13 \leq b2 \leq 19$ vorzugsweise $15 \leq b2 \leq 17$
iii. $0,05 \leq b3 \leq 0,15$ vorzugsweise $0,08 \leq a3 \leq 0,12$
iv. $0,9 \leq b4 \leq 1,4$ vorzugsweise $1,0 \leq b4 \leq 1,2$
v.

$$-4.4 \leq b5 \leq -4.1$$

vi. $2,4 \leq b6 \leq 2,7$ vorzugsweise $2,5 \leq b6 \leq 2,6$
vii. $6,0 \leq b7 \leq 6,4$ vorzugsweise $6,2 \leq b7 \leq 6,3$
viii. $-0,8 \leq b8 \leq -0,4$ vorzugsweise $-0,7 \leq b8 \leq -0,5$
ix.

$$1.0 \leq b9 \leq 1.1$$

x. $3,4 \leq b10 \leq 3,8$ vorzugsweise $3,6 \leq b10 \leq 3,7$
xi. $-5,0 \leq b11 \leq -4,5$ vorzugsweise $-4,9 \leq b11 \leq -4,8$
xii. $1,0 \leq b12 \leq 1,2$ vorzugsweise $1,05 \leq b12 \leq 1,15$.

5. *In* vitro-Verfahren nach Anspruch 4, wobei die logistische Funktion - 25,98652 + 16,00374 x Log(Alpha2Makroglobulin (g/l)) + 0,10067 x Alter (in Jahren) + 1,12881 x Log (ALT, IU / l) - 4,24187 x ApoA1 (g/l) + 2.55422 x Log(AST, IU / l) + 6.22308 x Log(Bilirubin ($\mu$mol/l)) + 0.59340 x Log(CT, mmol/l) + 1.07838 x Geschlecht (weiblich=0, männlich=1) + 3.64357 x Log(GGT (IU/l)) - 4.86167 x Log(Haptoglobin (g/l)) + 1.11641 Log (TG, mmol/l).

6. Verfahren zur Gewinnung einer logistischen Funktion, die in einem nichtinvasiven Diagnosetest zum Nachweis von NASH, nicht-alkoholischer Steato-Hepatitis, bei einem Patienten verwendet werden kann, wobei die logistische Funktion die Werte der Konzentration biochemischer Marker im Serum des Patienten kombiniert, umfassend die folgenden Schritte:

a) Einteilung von Patienten einer Patientenkohorte in verschiedene Gruppen entsprechend dem Vorhandensein von NASH, wie durch die Analyse einer Leberbiopsie bestimmt, wobei ein Patient als NASH-Patient eingestuft wird, wenn er

i. mindestens einen Faktor des metabolischen Syndroms und keine andere chronische oder akute Lebererkrankung aufweist und
ii. Hepatozyten-Ballonierung oder lobuläre Aktivität (jeweils mindestens Grad 1 oder mindestens Grad 2 bei einem)

b) unter den biochemischen Markern, deren Wert gemessen wurde, diejenigen zu identifizieren, die sich durch eindimensionale Analyse signifikant zwischen diesen Gruppen unterscheiden
c) Durchführung einer logistischen Regressionsanalyse zur Bewertung des unabhängigen Unterscheidungswerts dieser in Schritt b) identifizierten Marker für die Diagnose von NASH
d) die Konstruktion der logistischen Funktion durch Kombination dieser ermittelten unabhängigen Faktoren,

**dadurch gekennzeichnet, daß** die biochemischen Marker, deren Konzentration gemessen wird, ausgewählt werden aus der Gruppe bestehend aus $\alpha$2-Makroglobulin, AST (Aspartat-Aminotransferase), ALT (Alanin-Aminotransferase), GGT (Gamma-Glutamyl-Transpeptidase), Gesamtbilirubin, Haptoglobin, ApoA1, Triglyceride, Gesamt-cholesterin, $\gamma$-Globulin, Albumin, $\alpha$1-Globulin, $\alpha$2-Globulin, $\beta$-Globulin, IL10, TGF-$\beta$1, apoA2, apoB, Cytokeratin 18, Thrombozytenzahl, Prothrombinspiegel, Hyaluronsäure, Harnstoff, N-Terminus von Typ-III-Pro-Kollagen, Tissue Inhibitor Metalloproteinase Typ-1 (TIMP-1), Typ-IV-Kollagen (Coll IV) und Osteoprotegerin, wobei die logistische Funktion nicht BMI und Nüchternglukose umfasst,
wobei die logistische Funktion außerdem das Geschlecht und das Alter des Patienten enthält.

7. Verfahren nach Anspruch 6, wobei der Faktor des metabolischen Syndroms ausgewählt ist aus der Gruppe bestehend aus abdominaler Fettleibigkeit, hohen Serumtriglyceriden (ohne Behandlung), niedrigem High-Density-Lipoprotein (HDL)-Cholesterinspiegel (ohne Behandlung), erhöhtem Blutdruck und einem der Faktoren aus der Gruppe bestehend aus erhöhtem Nüchternplasmaglukose, Insulinresistenz und Prädiabetes.

**Revendications**

1. Méthode *in vitro* pour le diagnostic de la NASH, stéato-hépatite non alcoolique, chez un patient comprenant l'étape de :

    a) combiner les valeurs mesurées à partir des marqueurs présents dans le sérum ou le plasma dudit patient par le biais d'une fonction logistique, afin d'obtenir un indice, ledit indice devant être comparé à un indice de référence, la première fonction logistique étant a1 + a2 x Log (alpha2-macroglobuline (A2M), g/l) + a3 x Âge (années) + a4 x Log (alanine aminotransférase (ALT), IU / l) + a5 x (apolipoprotéine A-1 (Apoa1), g/l) + a6 x Log (Aspartate transaminase (AST), IU/l) + a7 x Log (bilirubine totale (BILI), μmol/l) + a8 x Log (cholestérol (CT), mmol/l) + a9 x sexe (0 pour les femmes, 1 pour les hommes) + a10 x log(gamma-glutamyl transpeptidase (GGT), UI / l) + a11 x log (haptoglobine (Hapto), g/l) + a12 x log (triglycérides (TG), mmol/l), dans laquelle

    i.

$$-8 \leq a1 \leq -7$$

    ii. $0{,}1 \leq a2 \leq 0{,}6$ de préférence $0{,}15 \leq a2 \leq 0{,}55$
    iii. $0{,}02 \leq a3 \leq 0{,}05$ de préférence $0{,}03 \leq a3 \leq 0{,}04$
    iv. $1{,}1 \leq a4 \leq 1{,}5$ de préférence $1{,}2 \leq a4 \leq 1{,}4$
    v.

$$-0{,}2 \leq a5 \leq 1{,}0$$

    vi. $1{,}8 \leq a6 \leq 2{,}3$ de préférence $1{,}95 \leq a6 \leq 2{,}2$
    vii. $0{,}8 \leq a7 \leq 1{,}6$ de préférence $0{,}9 \leq a7 \leq 1{,}5$
    viii. $-1{,}7 \leq a8 \leq -1{,}3$ de préférence $-1{,}6 \leq a8 \leq -1{,}4$
    ix.

$$0.015 \leq a9 \leq 0.20$$

    x. $0{,}15 \leq a10 \leq 0{,}25$ de préférence $0{,}20 \leq a10 \leq 0{,}22$
    xi.

$$-0.3 \leq a11 \leq 0.1$$

    xii. $0{,}9 \leq a12 \leq 1{,}2$ de préférence $1{,}0 \leq a12 \leq 1{,}1$.

2. Méthode *in vitro* selon la revendication 1, dans laquelle la fonction logistique est -7,82349 + 0,50879 x Log (A2M, g/l) + 0,036625 x Âge (années) + 1,22544 x Log (ALT, UI/l) -0,12954 x (Apoa1, g/l) + 2,18581 x Log (AST, UI/l) + 1. 48183 x Log (BILI, μmol/l) -1.49351 x Log (CT, mmol/l) + 0.019536 Sexe (0 pour les femmes, 1 pour les hommes) + 0.21614 x Log(GGT, IU / l) -0.026321 x Log (Hapto, g/l) + 1.09487 x Log (TG, mmol/l).

3. Méthode *in vitro* selon la revendication 1, dans laquelle la fonction logistique est -7,370196 + 0,18026 x Log (A2M, g/l) + 0,034609 x Age (années) + 1,47222 x Log (ALT, IU / l) + 0,089966 x (Apoa1, g/l) + 1,99317 x Log (AST, IU/l) + 0. 98523 x Log (BILI, μmol/l) -1,55580 x Log (CT, mmol/l) + 0,17857 x Sexe (0 pour les femmes, 1 pour les hommes) + 0,020437 x Log(GGT, UI / l) + 0,055873 x Log (Hapto, g/l) + 1,00712 x Log (TG, mmol/l).

4. Méthode *in vitro* pour le diagnostic de la gravité de la NAFLD (stéatose hépatique non alcoolique) chez un patient, comprenant les étapes suivantes :

    a) combiner les valeurs mesurées à partir des marqueurs présents dans le sérum ou le plasma dudit patient par le biais d'une fonction logistique, de manière à obtenir un indice, ledit indice devant être comparé à un indice de référence, ladite première fonction logistique étant b1 + b2 x Log (alpha2-macroglobuline (A2M), g/l) + b3 x Âge (années) + b4 x Log (alanine aminotransférase (ALT), IU / l) + b5 x (apolipoprotéine A-1 (Apoa1), g/l) + b6 x Log (Aspartate transaminase (AST), IU/l) + b7 x Log (bilirubine totale (BILI), $\mu$mol/l) + b8 x Log (cholestérol (CT), mmol/l) + b9 x Sexe (0 pour les femmes, 1 pour les hommes) + b10 x Log(gamma-glutamyl transpeptidase (GGT), IU / l) + b11 x Log (Haptoglobine (Hapto), g/l) + b12 x Log (Triglycérides (TG), mmol/l) afin d'obtenir un premier indice, dans laquelle

i. -28 ≤ b1 ≤ -22 de préférence -26 ≤ b1 ≤ -25
ii. 13 ≤ b2 ≤ 19 de préférence 15 ≤ b2 ≤ 17
iii. iii. 0,05 ≤ b3 ≤ 0,15 de préférence 0,08 ≤ a3 ≤ 0,12
iv. 0,9 ≤ b4 ≤ 1,4 de préférence 1,0 ≤ b4 ≤ 1,2
v.

$$-4.4 \leq b5 \leq -4.1$$

vi. 2,4 ≤ b6 ≤ 2,7 de préférence 2,5 ≤ b6 ≤ 2,6
vii. 6.0 ≤ b7 ≤ 6.4 de préférence 6.2 ≤ b7 ≤ 6.3
viii. -0,8 ≤ b8 ≤ -0,4 de préférence -0,7 ≤ b8 ≤ -0,5
ix.

$$1.0 \leq b9 \leq 1.1$$

x. 3,4 ≤ b10 ≤ 3,8 de préférence 3,6 ≤ b10 ≤ 3,7
xi. -5,0 ≤ b11 ≤ -4,5 de préférence -4,9 ≤ b11 ≤ -4,8
xii. 1,0 ≤ b12 ≤ 1,2 de préférence 1,05 ≤ b12 ≤ 1,15.

**5.** Méthode *in vitro* selon la revendication 4, dans laquelle la fonction logistique est - 25,98652 + 16,00374 x Log(Alpha2Macroglobuline (g/l)) + 0,10067 x Âge (en années) + 1,12881 x Log (ALT, IU / l) - 4,24187 x ApoA1 (g/l) + 2. 55422 x Log (AST, IU / l) + 6.22308 x Log(Bilirubine ($\mu$mol/l)) + 0.59340 x Log (CT, mmol/l) + 1.07838 x Sexe (femme=0, homme=1) + 3.64357 x Log(GGT (IU/l)) - 4.86167 x Log(Haptoglobine (g/l)) + 1.11641 Log (TG, mmol/l).

**6.** Méthode d'obtention d'une fonction logistique pouvant être utilisée dans un test de diagnostic non invasif pour détecter la NASH, stéato-hépatite non alcoolique, chez un patient, dans laquelle ladite fonction logistique combine les valeurs de la concentration des marqueurs biochimiques dans le sérum dudit patient, comprenant les étapes de :

a) classer les patients d'une cohorte de patients en différents groupes en fonction de la présence de NASH déterminée par l'analyse d'une biopsie du foie, un patient étant classé comme ayant une NASH s'il

i. présente au moins un facteur du syndrome métabolique et aucune autre maladie hépatique chronique ou aiguë, et
ii. présente un ballonnement des hépatocytes ou une activité lobulaire (au moins de grade 1 pour chacun ou au moins de grade 2 pour l'un d'entre eux).

b) identifier, parmi les marqueurs biochimiques dont la valeur a été mesurée, ceux qui diffèrent significativement entre ces groupes par une analyse unidimensionnelle
c) effectuer une analyse de régression logistique pour évaluer la valeur discriminante indépendante des marqueurs identifiés à l'étape b) pour le diagnostic de la NASH
d) construire la fonction logistique en combinant les facteurs indépendants identifiés,

dans laquelle les marqueurs biochimiques dont la concentration est mesurée sont choisis dans le groupe constitué de l'$\alpha$2-macroglobuline, de l'AST (aspartate aminotransférase), de l'ALT (alanine aminotransférase), de la GGT (gammaglutamyl transpeptidase), de la bilirubine totale, de l'haptoglobine, de l'apoA1, des triglycérides, du cholestérol total, de la $\gamma$-globuline, de l'albumine, de l'$\alpha$1-globuline, $\alpha$2-globuline, $\beta$-globuline, IL10, TGF-$\beta$1, apoA2, apoB, cytokératine 18, nombre de plaquettes, taux de prothrombine, acide hyaluronique, urée, N-terminal du pro-collagène de type III, inhibiteur tissulaire de la métalloprotéinase de type 1 (TIMP-1), collagène de type IV (Coll IV) et ostéoprotégérine, la fonction logistique ne comprenant pas l'IMC et la glycémie à jeun,

dans laquelle la fonction logistique comprend en outre le sexe et l'âge du patient.

7. Méthode selon la revendication 6, dans laquelle le facteur du syndrome métabolique est choisi dans le groupe constitué par l'obésité abdominale, un taux élevé de triglycérides sériques (sans traitement), un faible taux de cholestérol à lipoprotéines de haute densité (HDL) (sans traitement), une pression artérielle élevée et l'un du groupe constitué par une glycémie élevée à jeun, une résistance à l'insuline et un prédiabète.

Steatosis →Ballooning→Lobular Inflammation: Diagnosis

0 ──→ 0 ──────→ Steatosis
      1 ──────→ Steatosis
      2 ──────→ Steatosis

1, 2, 3

1 ──→ 0 ──────→ Steatosis
      1 ──────→ NASH
      2 ──────→ NASH

2 ──→ 0 ──────→ Steatosis
      1 ──────→ NASH
      2 ──────→ NASH

From Bedossa et al, 2014

Figure 1

| Features of SAF scoring system | | | NASH definitions | | | |
|---|---|---|---|---|---|---|
| Steatosis | Activity (SAF score) | | Original | Extensions | | |
| | | | | 1% | | |
| | | | | | | Steatosis not required |
| | | | | | | B2 or L2 accepted |
| | **Ballooning** | **Lobular Inflammation** | **V1-S5** | **V2-S1** | **V3-noS-B1L1** | **V4-noS-B0L2-B2L0** |
| **3 levels** | **3 levels** | **3 levels** | | | | |
| 0% | 0 | 0 | 0 | 0 | 0 | 0 |
| 0% | 0 | 1 | 0 | 0 | 0 | 0 |
| 0% | 0 | 2 | 0 | 0 | 0 | 1 |
| 0% | 1 | 0 | 0 | 0 | 0 | 0 |
| 0% | 1 | 1 | 0 | 0 | 1 | 1 |
| 0% | 1 | 2 | 0 | 0 | 1 | 1 |
| 0% | 2 | 0 | 0 | 0 | 0 | 1 |
| 0% | 2 | 1 | 0 | 0 | 1 | 1 |
| 0% | 2 | 2 | 0 | 0 | 1 | 1 |
| 1-4% | 0 | 0 | 0 | 0 | 0 | 0 |
| 1-4% | 0 | 1 | 0 | 0 | 0 | 0 |
| 1-4% | 0 | 2 | 0 | 0 | 0 | 1 |
| 1-4% | 1 | 0 | 0 | 0 | 0 | 0 |
| 1-4% | 1 | 1 | 0 | 1 | 1 | 1 |
| 1-4% | 1 | 2 | 0 | 1 | 1 | 1 |
| 1-4% | 2 | 0 | 0 | 0 | 0 | 1 |
| 1-4% | 2 | 1 | 0 | 1 | 1 | 1 |
| 1-4% | 2 | 2 | 0 | 1 | 1 | 1 |
| >=5% | 0 | 0 | 0 | 0 | 0 | 0 |
| >=5% | 0 | 1 | 0 | 0 | 0 | 0 |
| >=5% | 0 | 2 | 0 | 0 | 0 | 1 |
| >=5% | 1 | 0 | 0 | 0 | 0 | 0 |
| >=5% | 1 | 1 | 1 | 1 | 1 | 1 |
| >=5% | 1 | 2 | 1 | 1 | 1 | 1 |
| >=5% | 2 | 0 | 0 | 0 | 0 | 1 |
| >=5% | 2 | 1 | 1 | 1 | 1 | 1 |
| >=5% | 2 | 2 | 1 | 1 | 1 | 1 |

# Figure 2

Figure 3

## EP 3 513 193 B1

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006082522 A
- WO 2006103570 A
- WO 2002016949 A

**Non-patent literature cited in the description**

- **RATZIU et al.** *Aliment Pharmacol Ther*, 2007, vol. 25, 207-218
- **PERAZZO et al.** *Aliment Pharmacol Ther*, 2014, vol. 40, 1081-93
- **ANGULO et al.** *Hepatology*, April 2007, vol. 45 (4), 846-54
- **CICHOZ-LACH et al.** *Med Sci Monit.*, 2012, vol. 18 (12), CR735-CR740
- **HARRISON et al.** BARD scores equaling 0 or 1 are of high (96%) negative predictive value (NPV) for advanced fibrosis. *Gut*, 2008, vol. 57, 1441-47
- **BEDOSSA et al.** FLIP algorithm and SAF scoring system.. *Hepatology*, November 2012, vol. 56 (5), 1751-9
- *Hepatology*, August 2014, vol. 60 (2), 565-75
- **POYNARD et al.** *BMC Gastroenterol.*, 2006, vol. 6, 34
- **POYNARD et al.** *PLOS One*, 2012, vol. 7, e30325
- **BRUNT et al.** *Int. J. Mol. Sci.*, 2016, vol. 17, 97
- **BEDOSSA et al.** *Hepatology*, April 2014, vol. 60 (2), 565-75
- **KLEINER et al.** *Hepatology*, 2005, vol. 41, 1313-1321
- **BEDOSSA et al.** *Hepatology*, 2012
- **BEDOSSA et al.** *Hepatology*, 2014
- **RATZIU.** *Gastroenterology*, June 2005, vol. 128 (7), 1898-906
- **BEDOSSA.** *Hepatology*, 2012
- **KLEINER.** *Hepatology*, 2005
- **BEDOSSA et al.** *Hepatology.*, November 2012, vol. 56 (5), 1751-9
- **CASSIMAN D ; JAEKEN J.** NASH may be trash. *Gut*, 2008, vol. 57 (2), 141-4
- **FELDSTEIN et al.** Cytokeratin-18 fragment levels as noninvasive biomarker for nonalcoholic steatohepatitis: A multicenter validation study. *Hepatology*, 2009, vol. 50 (4), 1072-8
- **HALFON et al.** FibroTest-ActiTest as a non-invasive marker of liver fibrosis. *Gastroenterol Clin Biol.*, 2008, vol. 32 (6), 22-39
- **KLEINER et al.** Design and validation of a histological scoring system for nonalcoholic fatty liver disease.. *Hepatology*, 2005, vol. 41, 1313-1321
- **LIANGPUNSAKUL S ; CHALASANI N.** Is hypothyroidism a risk factor for non-alcoholic steatohepatitis?. *J Clin Gastroenterol.*, 2003, vol. 37 (4), 340-3
- **MUSSO et al.** Meta-analysis: Natural history of non-alcoholic fatty liver disease (NAFLD) and diagnostic accuracy of non-invasive tests for liver disease severity. *Annals of Medicine.*, 2010, vol. 43 (8), 1-33
- **RATZIU et al.** *Gastroenterology.*, June 2005, vol. 128 (7), 1898-906
- **RATZIU et al.** Diagnostic value of biochemical markers (FibroTest-FibroSURE) for the prediction of liver fibrosis in patients with non-alcoholic fatty liver disease. *BMC Gastroenterology*, 2006, vol. 6, 6
- **SOWA et al.** Novel algorithm for non-invasive assessment of fibrosis in NAFLD. *PLOS ONE*, 2013, vol. 8, 4
- **VUPPALANCHI R ; CHALASANI N.** Non-alcoholic fatty liver disease and non-alcoholic steatohepatitis: Selected practical issues in their evaluation and management. *Hepatology*, 2009, vol. 49 (1), 306-317